# EUROPEAN PATENT SPECIFICATION

(11) **EP 3 325 668 B1**
(45) Date of publication and mention of the grant of the patent: **06.01.2021**
(21) Application number: 16828612.8
(22) Date of filing: 22.07.2016
(51) Int. Cl.: C12N 15/10, C12N 15/62, C12N 9/22, A61K 31/713

(54) **EDITING MITOCHONDRIAL DNA**
EDITIEREN VON MITOCHONDRIALER DNA
ÉDITION DE L'ADN MITOCHONDRIAL

(30) Priority: 23.07.2015 US 201562195875 P; 29.01.2016 US 201662288614 P
(43) Date of publication of application: 30.05.2018
(73) Proprietor: Mayo Foundation for Medical Education and Research, Rochester, MN 55905 (US)
(72) Inventor: EKKER, Stephen C., Rochester, Minnesota 55906-2826 (US); CAMPBELL, Jarryd M., Rochester, Minnesota 55901-3879 (US)
(74) Representative: Fish & Richardson P.C.
(86) International application number: PCT/US2016/043576
(87) International publication number: WO 2017/015567

(56) References cited:
- US-A1- 2007 099 216
- US-A1- 2011 145 940
- US-A1- 2014 212 928
- US-A1- 2014 294 773
- US-A1- 2014 338 070
- US-B1- 6 395 523
- PRADEEP REDDY ET AL: "Selective Elimination of Mitochondrial Mutations in the Germline by Genome Editing", CELL, vol. 161, no. 3, 1 April 2015 (2015-04-01) , pages 459-469, XP055414794, AMSTERDAM, NL ISSN: 0092-8674, DOI: 10.1016/j.cell.2015.03.051
- P. A. GAMMAGE ET AL: "Mitochondrially targeted ZFNs for selective degradation of pathogenic mitochondrial genomes bearing large-scale deletions or point mutations", EMBO MOLECULAR MEDICINE, vol. 6, no. 4, 24 February 2014 (2014-02-24), pages 458-466, XP055545169, Weinheim ISSN: 1757-4676, DOI: 10.1002/emmm.201303672
- MICHAL MINCZUK ET AL: "Development of a single-chain, quasi-dimeric zinc-finger nuclease for the selective degradation of mutated human mitochondrial DNA", NUCLEIC ACIDS RESEARCH, vol. 36, no. 12, 29 May 2008 (2008-05-29), pages 3926-3938, XP055545180, ISSN: 0305-1048, DOI: 10.1093/nar/gkn313
- S R BACMAN ET AL: "Manipulation of mtDNA heteroplasmy in all striated muscles of newborn mice by AAV9-mediated delivery of a mitochondria-targeted restriction endonuclease", GENE THERAPY, vol. 19, no. 11, 1 December 2011 (2011-12-01), pages 1101-1106, XP055545137, GB ISSN: 0969-7128, DOI: 10.1038/gt.2011.196
- AREUM JO ET AL: "Efficient Mitochondrial Genome Editing by CRISPR/Cas9", BIOMED RESEARCH INTERNATIONAL, vol. 2015, no. 305716, 1 January 2015 (2015-01-01), pages 1-10, XP055403440, ISSN: 2314-6133, DOI: 10.1155/2015/305716
- RACHEK, LI ET AL.: 'Endonuclease III and endonuclease VIII conditionally targeted into mitochondria enhance mitochondrial DNA repair and cell survival following oxidative stress.' NUCLEIC ACIDS RESEARCH vol. 32, no. 10, 2004, pages 3240 - 3247, XP055350533
- REITMAN, ZJ ET AL.: 'Isocitrate Dehydrogenase 1 and 2 Mutations in Cancer: Alterations at a Crossroads of Cellular Metabolism.' J NATL CANCER INST vol. 102, no. 13, 2010, pages 932 - 941, XP002660975
- BITINAITE, J ET AL.: 'Fokl dimerization is required for DNA cleavage.' PROC. NATL. ACAD. SCI. USA. vol. 95, September 1998, pages 10570 - 10575, XP002212149

### SEQUENCE LISTING

<110> Mayo Foundation for Medical Education and Research
<120> EDITING MITOCHONDRIAL DNA
<130> 07039-1476WO1
<150> US 62/288,614
   <151> 2016-01-29
<150> US 62/195,875
   <151> 2015-07-23
<160> 106
<170> PatentIn version 3.5
<210> 1
   <211> 182
   <212> PRT
   <213> Artificial Sequence
<220>
   <223> synthetically generated polypeptide
<400> 1
   Met Ala Gly Tyr Leu Lys Val Leu Ser Ser Leu Ser Arg Ser Ala Ala
   1               5                   10                  15
   Thr Leu Ser Lys Ser Pro Ala Val Leu Ala Pro Ala Cys Gln Ser Leu
               20                  25                  30
   Gln Gln Arg Asn Tyr Ala Asp Lys Arg Ile Gln Ser Arg Asp Val Thr
           35                  40                  45
   Arg Val Asp Leu Arg Thr Leu Gly Tyr Ser Gln Gln Gln Gln Glu Lys
       50                  55                  60
   Ile Lys Pro Lys Val Arg Ser Thr Val Ala Gln His His Glu Ala Leu
   65                  70                  75                  80
   Val Gly His Gly Phe Thr His Ala His Ile Val Ala Leu Ser Gln His
                   85                  90                  95
   Pro Ala Ala Leu Gly Thr Val Ala Val Thr Tyr Gln His Ile Ile Thr
               100                 105                 110
   Ala Leu Pro Glu Ala Thr His Glu Asp Ile Val Gly Val Gly Lys Gln
           115                 120                 125
   Trp Ser Gly Ala Arg Ala Leu Glu Ala Leu Leu Thr Asp Ala Gly Glu
       130                 135                 140
   Leu Arg Gly Pro Pro Leu Gln Leu Asp Thr Gly Gln Leu Val Lys Ile
   145                 150                 155                 160
   Ala Lys Arg Gly Gly Val Thr Ala Met Glu Ala Val His Ala Ser Arg
                   165                 170                 175
   Asn Ala Leu Thr Gly Ala
               180
<210> 2
   <211> 242
   <212> PRT
   <213> Artificial Sequence
<220>
   <223> synthetically generated polypeptide
<400> 2
   His Leu Val Ala Leu Ala Cys Leu Gly Gly Arg Pro Ala Met Asp Ala
   1               5                   10                  15
   Val Lys Lys Gly Leu Pro His Ala Pro Glu Leu Ile Arg Arg Val Asn
               20                  25                  30
   Arg Arg Ile Gly Glu Arg Thr Ser His Arg Val Ala Ser Arg Ser Gln
           35                  40                  45
   Leu Val Lys Ser Glu Leu Glu Glu Lys Lys Ser Glu Leu Arg His Lys
       50                  55                  60
   Leu Lys Tyr Pro His Glu Tyr Ile Glu Leu Ile Glu Ile Ala Arg Asn
   65                  70                  75                  80
   Ser Thr Gln Asp Arg Ile Leu Glu Met Lys Val Met Glu Phe Phe Met
                   85                  90                  95
   Lys Val Tyr Gly Tyr Arg Gly Lys His Leu Gly Gly Ser Arg Lys Pro
               100                 105                 110
   Asp Gly Ala Ile Tyr Thr Val Gly Ser Pro Ile Asp Tyr Gly Val Ile
           115                 120                 125
   Val Asp Thr Lys Ala Tyr Ser Gly Gly Tyr Asn Leu Pro Ile Gly Gln
       130                 135                 140
   Ala Asp Glu Met Gln Arg Tyr Val Glu Glu Asn Gln Thr Arg Asn Lys
   145                 150                 155                 160
   His Ile Asn Pro Asn Glu Trp Trp Lys Val Tyr Pro Ser Ser Val Thr
                   165                 170                 175
   Glu Phe Lys Phe Leu Phe Val Ser Gly His Phe Lys Gly Asn Tyr Lys
               180                 185                 190
   Ala Gln Leu Thr Arg Leu Asn His Ile Thr Asn Cys Asn Gly Ala Val
           195                 200                 205
   Leu Ser Val Glu Glu Leu Leu Ile Gly Gly Glu Met Ile Lys Ala Gly
       210                 215                 220
   Thr Leu Thr Leu Glu Glu Val Arg Arg Lys Phe Asn Asn Gly Glu Ile
   225                 230                 235                 240
   Asn Phe
<210> 3
   <211> 175
   <212> DNA
   <213> Danio rerio
<400> 3
   atagtagttc ccaaagaatc accaattcga attttagttt cagctgaaga cgtactgcac 60
   tcttgagctg ttccatcatt aggcattaaa atagacgcgg tacccggacg attaaaccaa 120
   accgctttta ttgtttcacg tccaggtgta ttctacggac aatgctctga aatct 175
<210> 4
   <211> 175
   <212> DNA
   <213> Danio rerio
<400> 4
   atagtagttc ccaaagaatc accaattcga attttagttt cagctgaaga cgtactgcac 60
   tcttgagctg ttccatcatt aggcattaaa atagacgcgg tacccggacg attaaaccaa 120
   accgccttta ttgtttcacg tccaggtgta ttctacggac aatgctctga aatct 175
<210> 5
   <211> 96
   <212> DNA
   <213> Danio rerio
<400> 5
   atagtagttc ccaaagaatc accaattcga attttagttt cagctgaaga cgtactgcac 60
   tcttgagctg ttccatcatt aggcattaaa atagac 96
<210> 6
   <211> 28
   <212> DNA
   <213> Danio rerio
<400> 6
   atagtagttc ccaaagaatc accaattc 28
<210> 7
   <211> 175
   <212> DNA
   <213> Danio rerio
<400> 7
   atagtagttc ccaaagaatc accaattcga attttagctt cagctgaaga cgtactgcac 60
   tcttgagctg ttccatcatt aggcattaaa atagacgcgg tacccggacg attaaaccaa 120
   accgctttta ttgtttcacg tccaggtgta ttctacggac aatgctctga aatct 175
<210> 8
   <211> 175
   <212> DNA
   <213> Danio rerio
<400> 8
   ctaatcccta tacgaaaatt atttataggg gagtcagcag ccactaaaat tgaaaaatca 60
   tgaagcgaac tattcggacc atgcggaatt gcatttacat taataacagt ggccaccttt 120
   gttaaggacc atcgaatagc gtcaatcaag tcttacctag ccgtcttcct tacct 175
<210> 9
   <211> 93
   <212> DNA
   <213> Danio rerio
<400> 9
   gcggaattgc atttacatta ataacagtgg ccacctttgt taaggaccat cgaatagcgt 60
   caatcaagtc ttacctagcc gtcttcctta cct 93
<210> 10
   <211> 37
   <212> DNA
   <213> Danio rerio
<400> 10
   gcgtcaatca agtcttacct agccgtcctc cttacct 37
<210> 11
   <211> 91
   <212> DNA
   <213> Danio rerio
<400> 11
   ggaattgcat ttacattaat aacagtggcc acccttgtta aggaccatcg aatagcgtca 60
   atcaagtctt acctagccgt cttccttacc t 91
<210> 12
   <211> 91
   <212> DNA
   <213> Danio rerio
<400> 12
   ggaattgcat ttacattaat aacagtggcc acctttgtta aggaccatcg aatagcgtca 60
   atcaagtctt acctagccgt cttccttacc t 91
<210> 13
   <211> 140
   <212> DNA
   <213> Homo sapiens
<400> 13
   aagagaacca acacctcttt acagtgaaat gccccaacta aatactaccg tatggcccac      60
   cataattacc cccatactcc ttacactatt cctcatcacc caactaaaaa taataaacac     120
   aaactaccac ctacctccct                                                 140
<210> 14
   <211> 74
   <212> DNA
   <213> Homo sapiens
<400> 14
   aagagaacca acacctcttt acagtgaaat gccccaacta aatactaccg tatggcccac      60
   cataattacc ccca                                                        74
<210> 15
   <211> 36
   <212> DNA
   <213> Homo sapiens
<400> 15
   aagagaacca acacctcttt acagtgaaat gcccca 36
<210> 16
   <211> 35
   <212> DNA
   <213> Homo sapiens
<400> 16
   aagagaacca acacctcttt acagtgaaat gcccc 35
<210> 17
   <211> 158
   <212> DNA
   <213> Homo sapiens
<400> 17
   ctatctaggc cttcttacga gccaaaacct gcccctactc ctcctagacc taacctgact      60
   agaaaagcta ttacctaaaa caatttcaca gcaccaaatc tccacctcca tcatcacctc     120
   aacccaaaaa ggcataatta aactttactt cctctctt                             158
<210> 18
   <211> 28
   <212> DNA
   <213> Homo sapiens
<400> 18
   ggcataatta aactttactt cctctctt 28
<210> 19
   <211> 59
   <212> DNA
   <213> Homo sapiens
<400> 19
   ctccacctcc atcatcacct caacccaaaa aggcataatt aaactttact tcctctctt 59
<210> 20
   <211> 159
   <212> DNA
   <213> Homo sapiens
<400> 20
   tcaaaaaaat cgtagctttc tcaacatcaa gccaactagg gctaatgata gtagcaattg 60
   gcctaaacca accacatcta gcatttttcc acatctgcac tcacgctttt tttaaagcca 120
   tattattctt atgctcaggg gcaattattc acagtctta 159
<210> 21
   <211> 137
   <212> DNA
   <213> Homo sapiens
<400> 21
   aacatcaagc caactagggc taatgatagt agcaattggc ctaaaccaac cacatctagc 60
   atttttccac atctgcactc acgctttttt taaagccata ttattcttat gctcaggggc 120
   aattattcac agtctta 137
<210> 22
   <211> 109
   <212> DNA
   <213> Homo sapiens
<400> 22
   gtagcaattg gcctaaacca accacatcta gcatttttcc acatctgcac tcacgctttt 60
   tttaaagcca tattattctt atgctcaggg gcaattattc acagtctta 109
<210> 23
   <211> 102
   <212> DNA
   <213> Homo sapiens
<400> 23
   ttggcctaaa ccaaccacat ctagcatttt tccacatctg cactcacgct ttttttaaag        60
   ccatattatt cttatgctca ggggcaatta ttcacagtct ta                          102
<210> 24
   <211> 133
   <212> DNA
   <213> Homo sapiens
<400> 24
   tcaagccaac tagggctaat gatagtagca attggcctaa accaaccaca tctagcattt        60
   ttccacatct gcactcacgc tttttttaaa gccatattat tcttatgctc aggggcaatt       120
   attcacagtc tta                                                          133
<210> 25
   <211> 108
   <212> DNA
   <213> Homo sapiens
<400> 25
   tagcaattgg cctaaaccaa ccacatctag catttttcca catctgcact cacgcttttt        60
   ttaaagccat attattctta tgctcagggg caattattca cagtctta                    108
<210> 26
   <211> 159
   <212> DNA
   <213> Homo sapiens
<400> 26
   ttaccgacca cccttagtga atgcctcagc ttaatccaaa accctgattt ataatccttt        60
   tttctcatga gtcattttcc ttactattat tccaaccaaa atcattaatc acattcaacc       120
   taatgaccca actcaagttg atgctaaaga gcacaaaaa                              159
<210> 27
   <211> 138
   <212> DNA
   <213> Homo sapiens
<400> 27
   ttaccgacca cccttagtga atgcctcagc ttaatccaaa accctgattt ataatccttt        60
   tttctcatga gtcattttcc ttactattat tccaaccaaa atcattaatc acattcaacc       120
   taatgaccca actcaagt                                                     138
<210> 28
   <211> 133
   <212> DNA
   <213> Homo sapiens
<400> 28
   ttaccgacca cccttagtga atgcctcagc ttaatccaaa accctgattt ataatccttt 60
   tttctcatga gtcattttcc ttactattat tccaaccaaa atcattaatc acattcaacc 120
   taatgaccca act 133
<210> 29
   <211> 131
   <212> DNA
   <213> Homo sapiens
<400> 29
   ttaccgacca cccttagtga atgcctcagc ttaatccaaa accctgattt ataatccttt 60
   tttctcatga gtcattttcc ttactattat tccaaccaaa atcattaatc acattcaacc 120
   taatgaccca a 131
<210> 30
   <211> 102
   <212> DNA
   <213> Homo sapiens
<400> 30
   ttaccgacca cccttagtga atgcctcagc ttaatccaaa accctgattt ataatccttt 60
   tttctcatga gtcattttcc ttactattat tccaaccaaa at 102
<210> 31
   <211> 102
   <212> DNA
   <213> Homo sapiens
<400> 31
   ttaccgccca cccttagtga atgcttcagc ttaatccaaa accctgattt ataatccttt 60
   tttatcatga gtcattttcc taactattat tccaaccaaa at 102
<210> 32
   <211> 13
   <212> DNA
   <213> Homo sapiens
<400> 32
   ttaccgacca ccc 13
<210> 33
   <211> 12
   <212> DNA
   <213> Homo sapiens
<400> 33
   ttaccgacca cc 12
<210> 34
   <211> 9
   <212> PRT
   <213> Artificial Sequence
<220>
   <223> consensus
<400> 34
   Leu Ala Gly Leu Ile Asp Ala Asp Gly
   1               5
<210> 35
   <211> 15
   <212> DNA
   <213> Danio rerio
<400> 35
   agggctaatg atagt 15
<210> 36
   <211> 15
   <212> DNA
   <213> Danio rerio
<400> 36
   gctagatgtg gttgg 15
<210> 37
   <211> 15
   <212> DNA
   <213> Danio rerio
<400> 37
   tccttactat tattc 15
<210> 38
   <211> 15
   <212> DNA
   <213> Danio rerio
<400> 38
   taggttgaat gtgat 15
<210> 39
   <211> 15
   <212> DNA
   <213> Danio rerio
<400> 39
   tatattcgaa gctac 15
<210> 40
   <211> 15
   <212> DNA
   <213> Danio rerio
<400> 40
   cgggtaataa tgatt 15
<210> 41
   <211> 15
   <212> DNA
   <213> Danio rerio
<400> 41
   ttacacctaa ttcct 15
<210> 42
   <211> 15
   <212> DNA
   <213> Danio rerio
<400> 42
   aagttctggt tttaa 15
<210> 43
   <211> 15
   <212> DNA
   <213> Danio rerio
<400> 43
   gactaaaatg aacct 15
<210> 44
   <211> 15
   <212> DNA
   <213> Danio rerio
<400> 44
   acgagttaga agcat 15
<210> 45
   <211> 22
   <212> DNA
   <213> Artificial Sequence
<220>
   <223> synthetically generated primer
<400> 45
   ggtccgcccg cctaccattt tc 22
<210> 46
   <211> 27
   <212> DNA
   <213> Artificial Sequence
<220>
   <223> synthetically generated primer
<400> 46
   ccaggtgatg aataaggcga ttgaggt 27
<210> 47
   <211> 26
   <212> DNA
   <213> Artificial Sequence
<220>
   <223> synthetically generated primer
<400> 47
   gacgcggtac ccggacgatt aaacca 26
<210> 48
   <211> 27
   <212> DNA
   <213> Artificial Sequence
<220>
   <223> synthetically generated primer
<400> 48
   gcaataatgc ttcctcaggc aagccgt 27
<210> 49
   <211> 27
   <212> DNA
   <213> Artificial Sequence
<220>
   <223> synthetically generated primer
<400> 49
   aactcttggt gcaactccaa gtagaag 27
<210> 50
   <211> 21
   <212> DNA
   <213> Artificial Sequence
<220>
   <223> synthetically generated primer
<400> 50
   agcctacgcc gtaccagtat t 21
<210> 51
   <211> 21
   <212> DNA
   <213> Artificial Sequence
<220>
   <223> synthetically generated primer
<400> 51
   gtttcacgcc atcagctact g 21
<210> 52
   <211> 21
   <212> DNA
   <213> Artificial Sequence
<220>
   <223> synthetically generated primer
<400> 52
   gagagcgtct ataaggagta c 21
<210> 53
   <211> 21
   <212> DNA
   <213> Artificial Sequence
<220>
   <223> synthetically generated primer
<400> 53
   gagctcatca gaaacaggac t 21
<210> 54
   <211> 43
   <212> PRT
   <213> Danio rerio
<400> 54
   Met Ala Gly Tyr Leu Lys Val Leu Ser Ser Leu Ser Arg Ser Ala Ala
   1               5                   10                  15
   Thr Leu Ser Lys Ser Pro Ala Val Leu Ala Pro Ala Cys Gln Ser Leu
               20                  25                  30
   Gln Gln Arg Asn Tyr Ala Asp Lys Arg Ile Gln
           35                  40
<210> 55
   <211> 45
   <212> DNA
   <213> Danio rerio
<400> 55
   ttacacctaa ttcctatcat cttacttata ttaaaaccag aactt 45
<210> 56
   <211> 45
   <212> DNA
   <213> Danio rerio
<400> 56
   aagttctggt tttaatataa gtaagatgat aggaattagg tgtaa 45
<210> 57
   <211> 45
   <212> DNA
   <213> Danio rerio
<400> 57
   gactaaaatg aacctcggat acagggtgaa atgcttctaa ctcgt 45
<210> 58
   <211> 45
   <212> DNA
   <213> Danio rerio
<400> 58
   acgagttaga agcatttcac cctctatccg aggttcattt tagtc 45
<210> 59
   <211> 40
   <212> DNA
   <213> Artificial Sequence
<220>
   <223> synthetically generated oligonucleotide
<400> 59
   aaacagcttt ttaattgcct ttaaaaccag aacttatgtg 40
<210> 60
   <211> 40
   <212> DNA
   <213> Artificial Sequence
<220>
   <223> synthetically generated oligolnucleotide
<400> 60
   ctgtgaagca agtgccggcc ctcttaatga ctttacacct 40
<210> 61
   <211> 40
   <212> DNA
   <213> Artificial Sequence
<220>
   <223> synthetically generated oligonucleotide
<400> 61
   ataagtttat ccacagcccc ctcactcacc gaggaagagt 40
<210> 62
   <211> 40
   <212> DNA
   <213> Artificial Sequence
<220>
   <223> synthetically generated oligonucleotide
<400> 62
   gagcccaagg cggccttgaa aacatctctt aatgacttta 40
<210> 63
   <211> 40
   <212> DNA
   <213> Artificial Sequence
<220>
   <223> synthetically generated oligonucleotide
<400> 63
   cgaatgagcc caaggcggcc gagaacatct cttaatgact 40
<210> 64
   <211> 40
   <212> DNA
   <213> Artificial Sequence
<220>
   <223> synthetically generated oligonucleotide
<400> 64
   cgaatgagcc caaggcggcc catctcttaa tgactttaca 40
<210> 65
   <211> 40
   <212> DNA
   <213> Artificial Sequence
<220>
   <223> synthetically generated oligonucleotide
<400> 65
   cgaatgagcc caaggcggcc atctcttaat gactttacac 40
<210> 66
   <211> 40
   <212> DNA
   <213> Artificial Sequence
<220>
   <223> synthetically generated oligonucleotide
<400> 66
   cgaatgagcc caaggcggcc tctcttaatg actttacacc 40
<210> 67
   <211> 40
   <212> DNA
   <213> Artificial Sequence
<220>
   <223> synthetically generated oligonucleotide
<400> 67
   tgcctttatt agcttaacat gacctaagtt ttaaatttga 40
<210> 68
   <211> 40
   <212> DNA
   <213> Artificial Sequence
<220>
   <223> synthetically generated oligonucleotide
<400> 68
   gcctgtgaag caagtgccgg cccttattaa aaccaaaaag 40
<210> 69
   <211> 45
   <212> DNA
   <213> Danio rerio
<400> 69
   agggctaatg atagtagcaa ttggcctaaa ccaaccacat ctagc 45
<210> 70
   <211> 45
   <212> DNA
   <213> Danio rerio
<400> 70
   gctagatgtg gttggtttag gccaattgct actatcatta gccct 45
<210> 71
   <211> 45
   <212> DNA
   <213> Danio rerio
<400> 71
   tccttactat tattccaacc aaaatcatta atcacattca accta 45
<210> 72
   <211> 45
   <212> DNA
   <213> Danio rerio
<400> 72
   taggttgaat gtgattaatg attttggttg gaataatagt aagga 45
<210> 73
   <211> 40
   <212> DNA
   <213> Artificial Sequence
<220>
   <223> synthetically generated oligonucleotide
<400> 73
   ttcaacctaa tgacccaact aacatcaagc caactagggc 40
<210> 74
   <211> 40
   <212> DNA
   <213> Artificial Sequence
<220>
   <223> synthetically generated oligonucleotide
<400> 74
   cctaatgacc caactcaagt gtagcaattg gcctaaacca 40
<210> 75
   <211> 40
   <212> DNA
   <213> Artificial Sequence
<220>
   <223> synthetically generated oligonucleotide
<400> 75
   actattattc caaccaaaat aacatcaagc caactagggc 40
<210> 76
   <211> 40
   <212> DNA
   <213> Artificial Sequence
<220>
   <223> synthetically generated oligonucleotide
<400> 76
   actattattc caaccaaaat gtagcaattg gcctaaacca 40
<210> 77
   <211> 40
   <212> DNA
   <213> Artificial Sequence
<220>
   <223> synthetically generated oligonucleotide
<400> 77
   cttggtgctt accgaccacc tcaagccaac tagggctaat 40
<210> 78
   <211> 40
   <212> DNA
   <213> Artificial Sequence
<220>
   <223> synthetically generated oligonucleotide
<400> 78
   ttggtgctta ccgaccaccc ttggcctaaa ccaaccacat 40
<210> 79
   <211> 40
   <212> DNA
   <213> Artificial Sequence
<220>
   <223> synthetically generated oligonucleotide
<400> 79
   ttggtgctta ccgaccaccc gtagcaattg gcctaaacca 40
<210> 80
   <211> 40
   <212> DNA
   <213> Artificial Sequence
<220>
   <223> synthetically generated oligonucleotide
<400> 80
   cattcaacct aatgacccaa catcgggaaa atagccctta 40
<210> 81
   <211> 40
   <212> DNA
   <213> Artificial Sequence
<220>
   <223> synthetically generated oligonucleotide
<400> 81
   aatgctctga aatctgcggg tagcaattgg cctaaaccaa 40
<210> 82
   <211> 40
   <212> DNA
   <213> Artificial Sequence
<220>
   <223> synthetically generated oligonucleotide
<400> 82
   aatgctctga aatctgcggg gcctaaacca accacatcta 40
<210> 83
   <211> 45
   <212> DNA
   <213> Danio rerio
<400> 83
   tatattcgaa gctacactta tcccaacact aatcattatt acccg 45
<210> 84
   <211> 45
   <212> DNA
   <213> Danio rerio
<400> 84
   cgggtaataa tgattagtgt tgggataagt gtagcttcga atata 45
<210> 85
   <211> 40
   <212> DNA
   <213> Artificial Sequence
<220>
   <223> synthetically generated oligonucleotide
<400> 85
   caatgctctg aaatctgcgg gtagcaattg gcctaaacca 40
<210> 86
   <211> 40
   <212> DNA
   <213> Artificial Sequence
<220>
   <223> synthetically generated oligonucleotide
<400> 86
   cctaatgacc caactcaagt ccgcagttta tagcttccga 40
<210> 87
   <211> 40
   <212> DNA
   <213> Artificial Sequence
<220>
   <223> synthetically generated oligonucleotide
<400> 87
   actattattc caaccaaaat gcaggatttt tctcaaaaga 40
<210> 88
   <211> 40
   <212> DNA
   <213> Artificial Sequence
<220>
   <223> synthetically generated oligonucleotide
<400> 88
   aaaccctgat ttataatcct gtagcaattg gcctaaacca 40
<210> 89
   <211> 40
   <212> DNA
   <213> Artificial Sequence
<220>
   <223> synthetically generated oligonucleotide
<400> 89
   ttggtgctta ccgaccaccc ccttccttgc aggatttttc 40
<210> 90
   <211> 40
   <212> DNA
   <213> Artificial Sequence
<220>
   <223> synthetically generated oligonucleotide
<400> 90
   aaacttggtg cttaccgacc aagccaacta gggctaatga 40
<210> 91
   <211> 40
   <212> DNA
   <213> Artificial Sequence
<220>
   <223> synthetically genearted oligonucleotide
<400> 91
   ttggtgctta ccgaccaccc aagccaacta gggctaatga 40
<210> 92
   <211> 136
   <212> DNA
   <213> Homo sapiens
<400> 92
   caagcgccta tagcactcga ataattcttc tcaccctaac aggtcaacct cgcttcccca 60
   cccttactaa cattaacgaa aataacccca ccctactaaa ccccattaaa cgcctggcag 120
   ccggaagcct attcgc 136
<210> 93
   <211> 94
   <212> DNA
   <213> Homo sapiens
<400> 93
   gtcaacctcg cttccccacc cttactaaca ttaacgaaaa taaccccacc ctactaaacc 60
   ccattaaacg cctggcagcc ggaagcctat tcgc 94
<210> 94
   <211> 103
   <212> DNA
   <213> Homo sapiens
<400> 94
   ttcatgccca tcgtcctaga attaattccc ctaaaaatct ttgaaatagg gcccgtattt 60
   accctatagc accccctcta ccccctctag agcccactgt aaa 103
<210> 95
   <211> 45
   <212> DNA
   <213> Homo sapiens
<400> 95
   attcctcatc acccaactaa aaatattaaa cacaaactac cacct 45
<210> 96
   <211> 55
   <212> DNA
   <213> Homo sapiens
<400> 96
   ttcatgccca tcgtcctaga attaattccc ctaaaaatct ttgaaatagg gcccg 55
<210> 97
   <211> 242
   <212> PRT
   <213> Artificial Sequence
<220>
   <223> synthetically generated polypeptide
<400> 97
   His Leu Val Ala Leu Ala Cys Leu Gly Gly Arg Pro Ala Met Asp Ala
   1               5                   10                  15
   Val Lys Lys Gly Leu Pro His Ala Pro Glu Leu Ile Arg Arg Val Asn
               20                  25                  30
   Arg Arg Ile Gly Glu Arg Thr Ser His Arg Val Ala Ser Arg Ser Gln
           35                  40                  45
   Leu Val Lys Ser Glu Leu Glu Glu Lys Lys Ser Glu Leu Arg His Lys
       50                  55                  60
   Leu Lys Tyr Pro His Glu Tyr Ile Glu Leu Ile Glu Ile Ala Arg Asn
   65                  70                  75                  80
   Ser Thr Gln Asp Arg Ile Leu Glu Met Lys Val Met Glu Phe Phe Met
                   85                  90                  95
   Lys Val Tyr Gly Tyr Arg Gly Lys His Leu Gly Gly Ser Arg Lys Pro
               100                 105                 110
   Asp Gly Ala Ile Tyr Thr Val Gly Ser Pro Ile Asp Tyr Gly Val Ile
           115                 120                 125
   Val Asp Thr Lys Ala Tyr Ser Gly Gly Tyr Asn Leu Pro Ile Gly Gln
       130                 135                 140
   Ala Ala Glu Met Gln Arg Tyr Val Glu Glu Asn Gln Thr Arg Asn Lys
   145                 150                 155                 160
   His Ile Asn Pro Asn Glu Trp Trp Lys Val Tyr Pro Ser Ser Val Thr
                   165                 170                 175
   Glu Phe Lys Phe Leu Phe Val Ser Gly His Phe Lys Gly Asn Tyr Lys
               180                 185                 190
   Ala Gln Leu Thr Arg Leu Asn His Ile Thr Asn Cys Asn Gly Ala Val
           195                 200                 205
   Leu Ser Val Glu Glu Leu Leu Ile Gly Gly Glu Met Ile Lys Ala Gly
       210                 215                 220
   Thr Leu Thr Leu Glu Glu Val Arg Arg Lys Phe Asn Asn Gly Glu Ile
   225                 230                 235                 240
   Asn Phe
<210> 98
   <211> 242
   <212> PRT
   <213> Artificial Sequence
<220>
   <223> synthetically generated polypeptide
<400> 98
   His Leu Val Ala Leu Ala Cys Leu Gly Gly Arg Pro Ala Met Asp Ala
   1               5                   10                  15
   Val Lys Lys Gly Leu Pro His Ala Pro Glu Leu Ile Arg Arg Val Asn
               20                  25                  30
   Arg Arg Ile Gly Glu Arg Thr Ser His Arg Val Ala Ser Arg Ser Gln
           35                  40                  45
   Leu Val Lys Ser Glu Leu Glu Glu Lys Lys Ser Glu Leu Arg His Lys
       50                  55                  60
   Leu Lys Tyr Pro His Glu Tyr Ile Glu Leu Ile Glu Ile Ala Arg Asn
   65                  70                  75                  80
   Ser Thr Gln Asp Arg Ile Leu Glu Met Lys Val Met Glu Phe Phe Met
                   85                  90                  95
   Lys Val Tyr Gly Tyr Arg Gly Lys His Leu Gly Gly Ser Arg Lys Pro
               100                 105                 110
   Ala Gly Ala Ile Tyr Thr Val Gly Ser Pro Ile Asp Tyr Gly Val Ile
           115                 120                 125
   Val Asp Thr Lys Ala Tyr Ser Gly Gly Tyr Asn Leu Pro Ile Gly Gln
       130                 135                 140
   Ala Asp Glu Met Gln Arg Tyr Val Glu Glu Asn Gln Thr Arg Asn Lys
   145                 150                 155                 160
   His Ile Asn Pro Asn Glu Trp Trp Lys Val Tyr Pro Ser Ser Val Thr
                   165                 170                 175
   Glu Phe Lys Phe Leu Phe Val Ser Gly His Phe Lys Gly Asn Tyr Lys
               180                 185                 190
   Ala Gln Leu Thr Arg Leu Asn His Ile Thr Asn Cys Asn Gly Ala Val
           195                 200                 205
   Leu Ser Val Glu Glu Leu Leu Ile Gly Gly Glu Met Ile Lys Ala Gly
       210                 215                 220
   Thr Leu Thr Leu Glu Glu Val Arg Arg Lys Phe Asn Asn Gly Glu Ile
   225                 230                 235                 240
   Asn Phe
<210> 99
   <211> 15
   <212> DNA
   <213> Homo sapiens
<400> 99
   attcctcatc accca 15
<210> 100
   <211> 15
   <212> DNA
   <213> Homo sapiens
<400> 100
   aggtggtagt ttgtg 15
<210> 101
   <211> 15
   <212> DNA
   <213> Homo sapiens
<400> 101
   ctcaccctaa caggt 15
<210> 102
   <211> 15
   <212> DNA
   <213> Homo sapiens
<400> 102
   aatgttagta agggt 15
<210> 103
   <211> 15
   <212> DNA
   <213> Homo sapiens
<400> 103
   cacaatcatg gcaag 15
<210> 104
   <211> 15
   <212> DNA
   <213> Homo sapiens
<400> 104
   gatagtggtt cactg 15
<210> 105
   <211> 27
   <212> DNA
   <213> Artificial Sequence
<220>
   <223> synthetically generated oligonucleotide
<400> 105
   ctcatgagct gtccccacat taggctt 27
<210> 106
   <211> 27
   <212> DNA
   <213> Artificial Sequence
<220>
   <223> synthetically generated oligonucleotide
<400> 106
   gcgatggagg taggattggt gctgtgg 27

## Description

### TECHNICAL FIELD

This document relates to materials and methods for making targeted changes to mitochondrial DNA.

### BACKGROUND

Mitochondria are an essential component of eukaryotic cells, and deficiencies in their function are associated with diverse diseases. A greater understanding of how changes in mitochondrial DNA (mtDNA) affect eukaryotic systems has been difficult, at least in part because of the limited options for manipulating the mtDNA genome.

P. Reddy et al. describe in Cell, vol. 161, no. 3, 1 April 2015, on pages 459-469 a selective elimination of mitochondrial mutations in the germline by genome editing.

P. A. Gammage et al. describe in EMBO Molecular Medicine, vol. 6, no. 4, 24 February 2014, on pages 458-466 mitochondrially targeted zinc finger nucleases for selective degradation of pathogenic mitochondrial genomes bearing large-scale deletions or point mutations.

M. Minczuk et al. describe in Nucleic Acids Research, vol. 36, no. 12, 29 May 2008, on pages 3926-3938 a development of a single-chain, quasi-dimeric zinc-finger nuclease for the selective degradation of mutated human mitochondrial DNA.

S. R. Bracman et al. describe in Gene Therapy, vol. 19, no. 11, 1 December 2011, on pages 1101-1106 a manipulation of mtDNA heteroplasmy in all striated muscles of newborn mice by AAV9-medlated delivery of a mitochondria- targeted restriction endonuclease.

A. Jo et al. describe in Biomed Research International, vol. 2015, no. 305716, 1 January 2015, on pages 1-10 an efficient mitochondrial genome editing by CRISPR/Cas9.

US 2014/212928 A1 describes methods and compositions for generating a single-stranded break in a target sequence, which facilitates targeted integration of one or more exogenous sequences.

US 6,395,523 B1 describes methods to engineer nicking endonucleases from Type II restriction endonucleases.

### SUMMARY

The present invention is defined by the independent claims. The dependent claims depict additional embodiments of the invention.

This document is based, at least in part, on the discovery that targeted deletions in can be made within the mtDNA genome using sequence-specific DNA endonucleases (e.g., attenuated transcription activator-like effector nucleases) that cause breaks in the DNA. When fused to a programmable DNA binding domain, these sequence-specific DNA endonucleases can be targeted to any DNA sequence. As described herein, by targeting this site-directed system to mitochondrial DNA, targeted deletions of about 5 kb were successfully generated *in vitro* in human cell culture, and targeted deletions of about 5 and 6 kb were generated *in vivo* in *Dania rerio* (zebrafish). DNA sequence analysis revealed that the deletions were specific to the targeted endonucleases, and were stable in both *in vitro* and *in vivo* systems. This is the first demonstration of DNA editing specific for mitochondria.

In a first aspect, this document features an in vitro method for modifying mitochondrial DNA within a cell, comprising introducing into the cell a recombinant DNA endonuclease targeted to a selected sequence within the mitochondrial DNA. The method further includes introducing into the cell a second recombinant DNA endonuclease targeted to a second selected sequence within the mitochondrial DNA, wherein the first DNA endonuclease and the second DNA endonuclease are targeted to different sequences within the mitochondrial DNA. The method is not a process for modifying the germ line genetic identity of human beings. The recombinant DNA endonuclease can have a mutation that results in attenuated endonuclease activity, as compared to a corresponding DNA endonuclease that lacks the mutation. The recombinant DNA endonuclease can have nickase activity. The DNA endonuclease can include (a) a first portion containing a mitochondrial targeting sequence, (b) a second portion containing an amino acid sequence targeting the DNA endonuclease to the selected sequence within the mitochondrial DNA, and (c) a third portion containing a nuclease domain that cleaves the mitochondrial DNA at or near the selected sequence. The first portion can include an isocitrate dehydrogenase 2 mitochondrial targeting sequence. The second portion can include a transcription activator-like effector (TALE) backbone and a plurality of tandem repeat sequences comprising repeat variable dinucleotides that, in combination, are targeted to the selected mitochondrial DNA sequence. The nuclease domain can be from a modified *Fok*I endonuclease, or a portion thereof (e.g., a *Fok*I endonuclease or portion thereof having an aspartic acid to alanine substitution at the amino acid position corresponding to position 483 or position 450 of an unmodified *Fok*I endonuclease). The introducing can include transforming a nucleic acid encoding the DNA endonuclease into the cell.

In another aspect, this document features a recombinant polypeptide that includes (a) a first portion containing a mitochondrial targeting sequence, (b) a second portion containing an amino acid sequence targeting the polypeptide to a selected mitochondrial DNA sequence, and (c) a third portion containing a nuclease domain that cleaves mitochondrial DNA at or near the selected sequence. The first portion contains an isocitrate dehydrogenase 2 mitochondrial targeting sequence. The second portion can contain a TALE backbone and a plurality of tandem repeat sequences comprising repeat variable dinucleotides that, in combination, are targeted to the selected mitochondrial DNA sequence. The nuclease domain can be from a modified *Fok*I endonuclease, or a portion thereof (e.g., a *Fok*I endonuclease or portion thereof having an aspartic acid to alanine substitution at the amino acid position corresponding to position 483 or position 450 of an unmodified *Fok*I endonuclease).

This document also features a nucleic acid comprising a nucleotide sequence that encodes a chimeric DNA endonuclease polypeptide as described herein, as well as a vector containing the nucleic acid, and a host cell containing the vector. The cell is not the human body, at the various stages of its formation and development.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict between the present specification and a reference mentioned herein, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF DRAWINGS

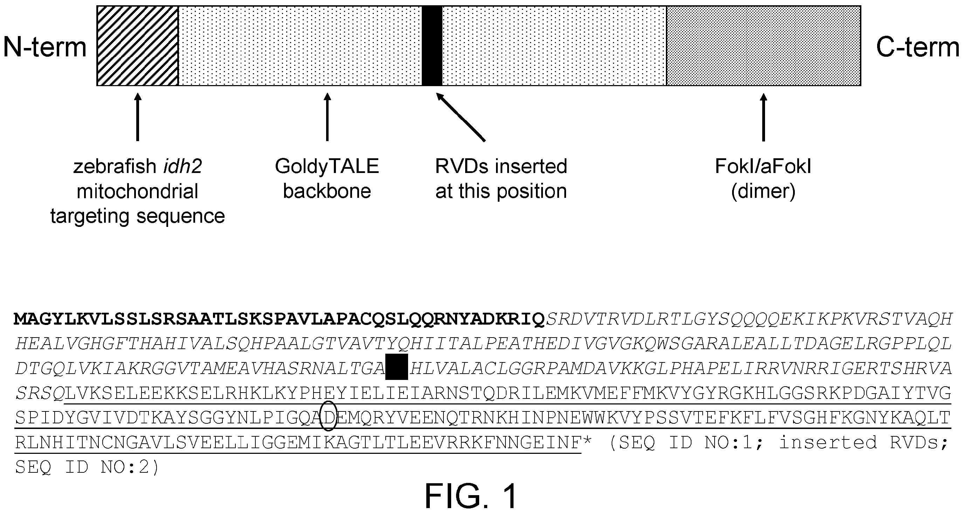

FIG. 1 is a diagram showing the structure (top) and sequence (bottom; SEQ ID NOS:1 and 2) of a heterodimeric a-mitoTALE nuclease protein. The protein includes a zebrafish *idh2* mitochondrial targeting sequence (hatched box in structure, bold text in sequence), a GoldyTALE backbone (dotted box, italics in sequence) with sequences containing repeat variable diresidues (RVDs) inserted (black boxes in structure and text), and a *Fok*I/a*Fok*I dimer sequence (gray box in structure, underlined text in sequence). The circled aspartic acid at position 483 of the *Fok*I sequence indicates the position of an Asp to Ala mutation in the a*Fok*I portion of the protein. The sequence with the Asp to Ala mutation is set forth in SEQ ID NO:97.

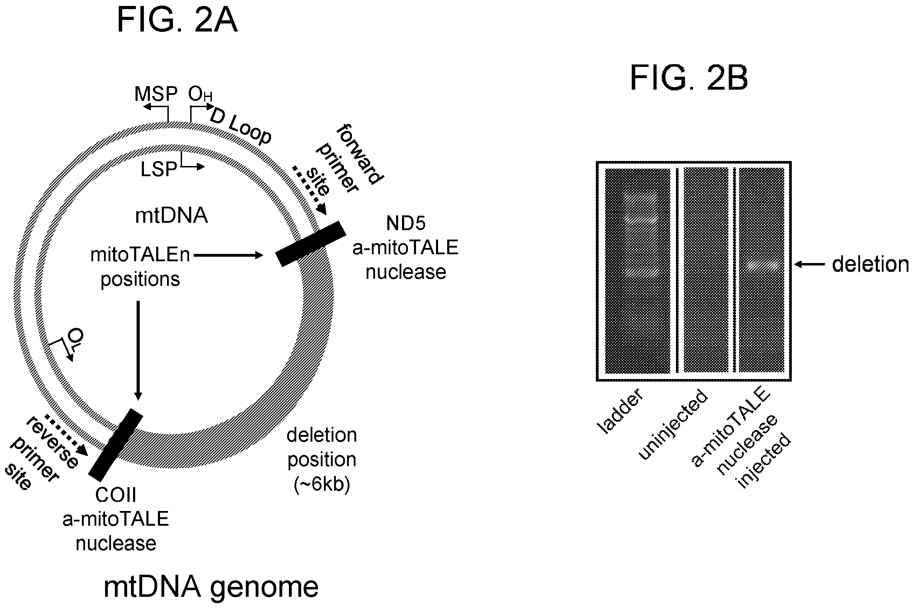

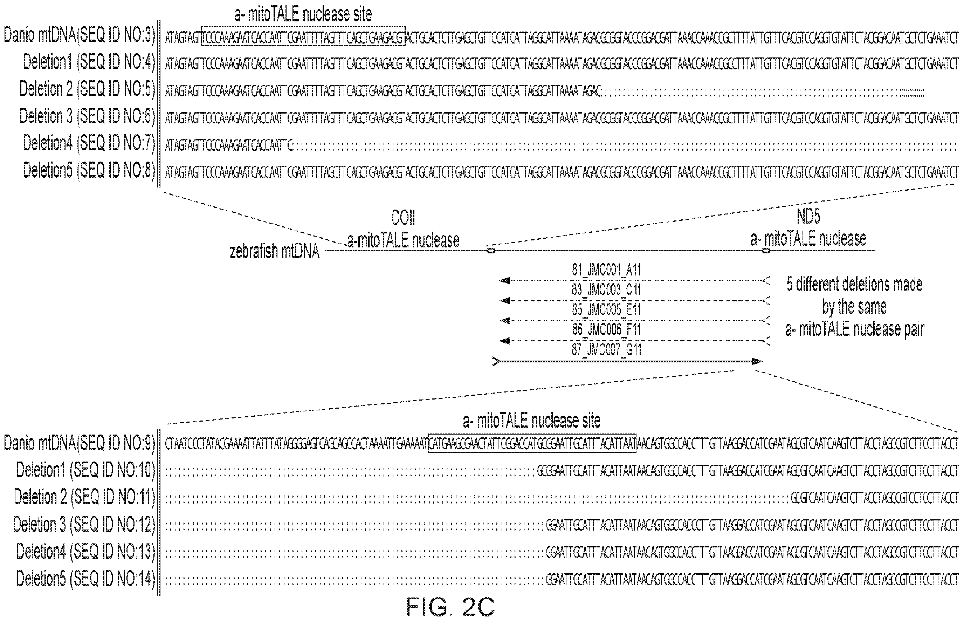

FIGS. 2A-2C show that mtDNA deletions can be generated in zebrafish using a-mitoTALE nucleases. FIG. 2A is a diagram of a zebrafish mitochondrial genome, indicating the locations of PCR primer sites and a-mitoTALE nuclease-generated deletions. FIG. 2B is a picture of an agarose gel containing PCR products from zebrafish injected with a-mitoTALE nucleases targeted to *nd5* and *coII* (right lane), or from uninjected zebrafish (center lane). Injection of the a-mitoTALE nucleases resulted in a deletion in the mtDNA. FIG. 2C shows the sequences of zebrafish mtDNAs containing five different deletions made by the same pair of a-mitoTALE nucleases, confirming the starting and ending positions of the deletions within the mtDNA genome. Target sites for the a-mitoTALE nucleases are boxed. Danio mtDNA genome sequence, SEQ ID NOS:3 and 8; deletion 1, SEQ ID NOS:4 and 9; deletion 2, SEQ ID NOS:5 and 10; deletion 3, SEQ ID NOS:3 and 11; deletion 4, SEQ ID NOS:6 and 12; deletion 5, SEQ ID NOS:7 and 12.

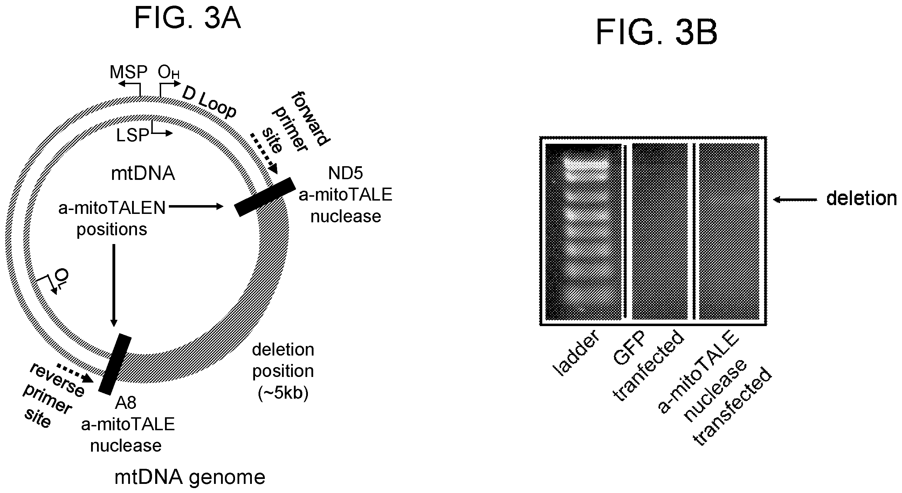

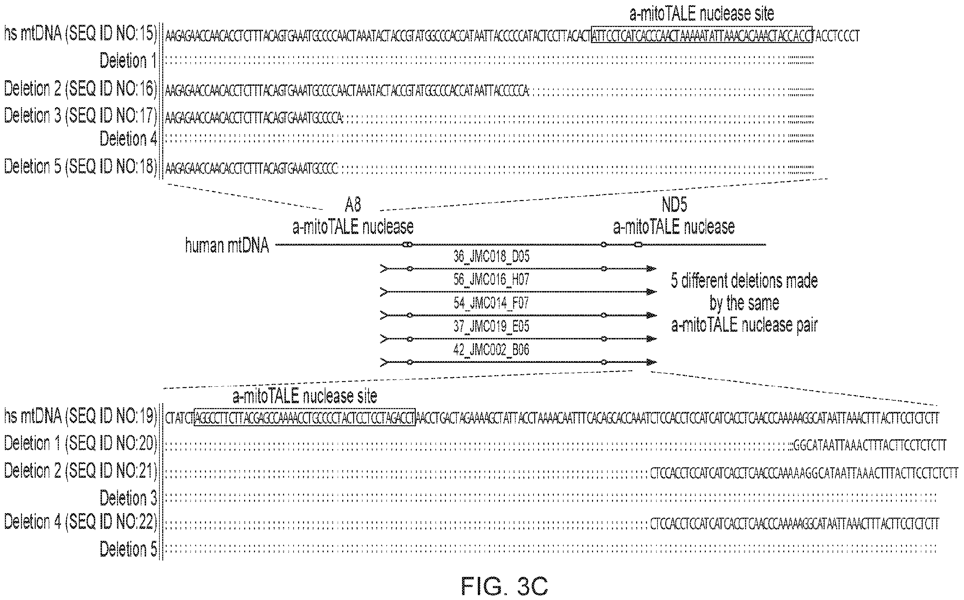

FIGS. 3A-3C show that mtDNA deletions can be generated in cultured human cells using a-mitoTALE nucleases. FIG. 3A is a diagram of a human mitochondrial genome, indicating the locations of PCR primer sites and a-mitoTALE nuclease-mediated deletions. FIG. 3B is a picture of an agarose gel containing PCR products from cells injected with a-mitoTALE nucleases targeted to ND5 and A8 (right lane), or from uninjected cells (center lane). Injection of the a-mitoTALE nucleases resulted in deletions in the mtDNA. FIG. 3C shows the sequences of mtDNAs containing five different deletions made by the same pair of a-mitoTALE nucleases, confirming the starting and ending positions of the deletions within the mtDNA genome. Target sites for the a-mitoTALE nucleases are boxed. Human mtDNA genome sequence, SEQ ID NOS:13 and 17; deletion 1, complete deletion and SEQ ID NO:18; deletion 2, SEQ ID NOS:14 and 19; deletion 3, SEQ ID NO: 15 and complete deletion; deletion 4, complete deletion and SEQ ID NO: 19; deletion 5, SEQ ID NO: 16 and complete deletion.

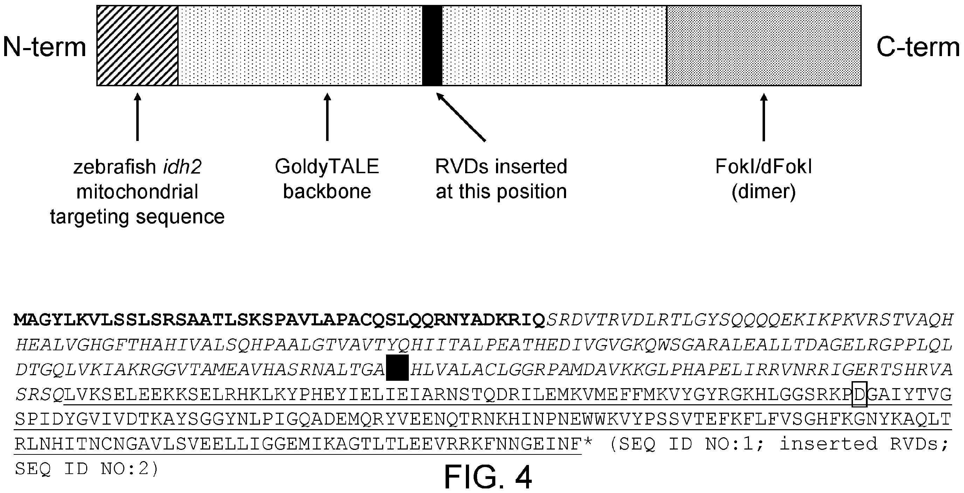

FIG. 4 is a diagram showing the structure (top) and sequence (bottom; SEQ ID NOS:1 and 2) of a heterodimeric d-mitoTALE nickase protein. The protein includes a zebrafish *idh2* mitochondrial targeting sequence (hatched box in structure, bold text in sequence), a GoldyTALE backbone (dotted box, italics in sequence) with sequences containing repeat variable diresidues (RVDs) inserted (black boxes in structure and text), and a *Fok*I/d*Fok*I dimer sequence (gray box in structure, underlined text in sequence). The boxed aspartic acid at position 450 of the *Fok*I sequence indicates the position of an Asp to Ala mutation in the d*Fok*I portion of the protein, which renders the *Fok*I endonuclease domain catalytically inactive. The sequence with the Asp to Ala mutation is set forth in SEQ ID NO:98.

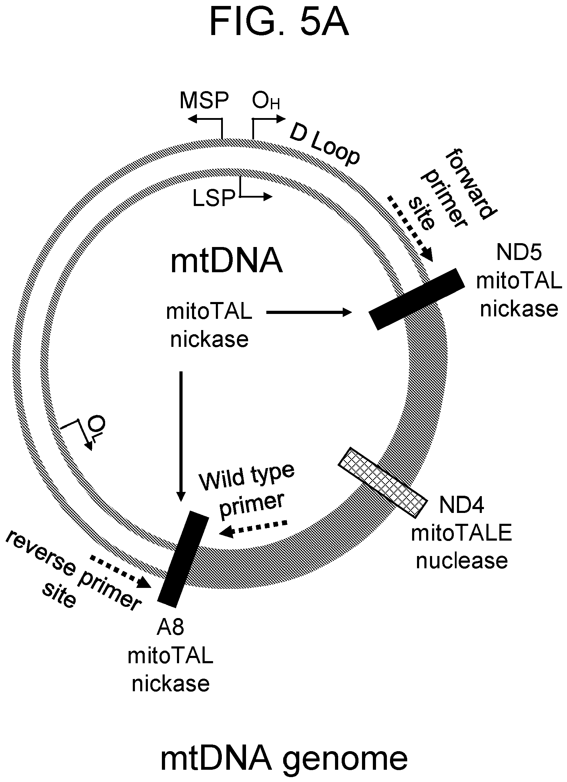

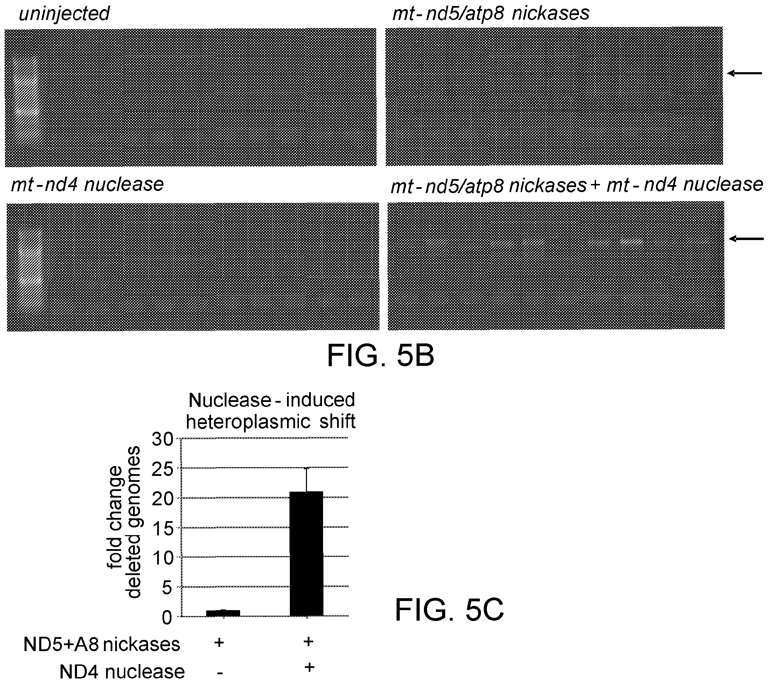

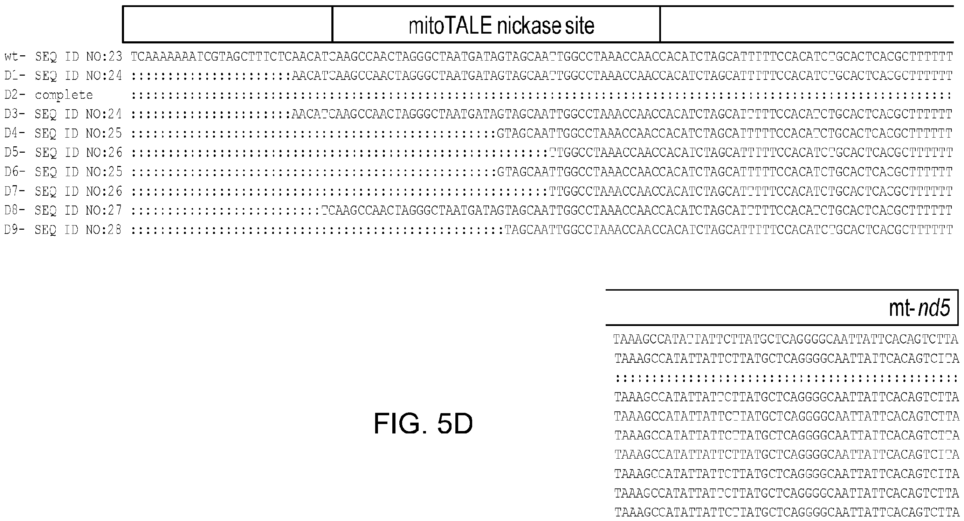

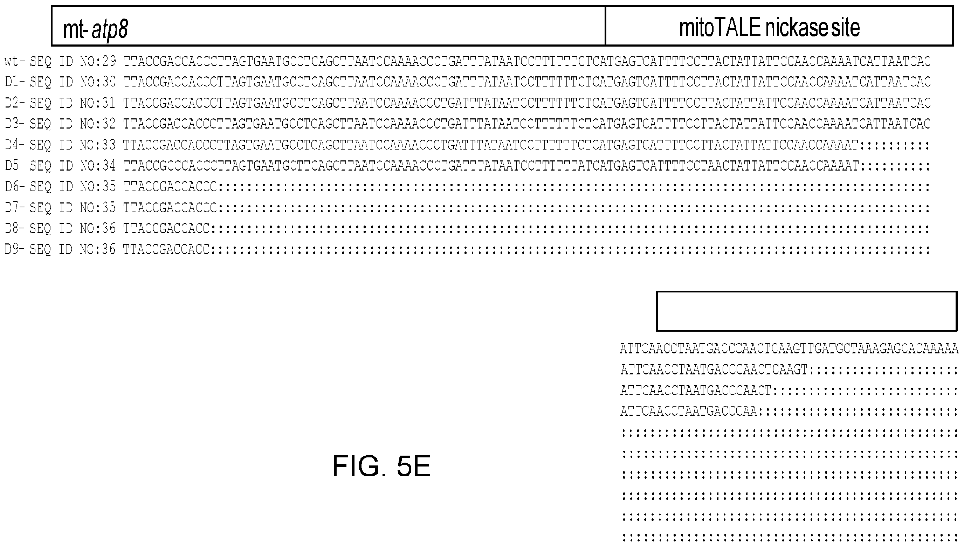

FIGS. 5A-5E show that simultaneous delivery of a mitoTALE endonuclease with mitoTALE nickases can increase the number of deleted mtDNA genomes as compared to mitoTALE nickases alone. FIG. 5A is a diagram of a zebrafish mitochondrial genome, indicating the locations of PCR primer sites and mitoTALE nickase-mediated deletions. FIG. 5B is a series of pictures of agarose gels containing PCR products from cells injected with mitoTALE nickases targeted to *nd5* and *atp8* (top right panel), a mitoTALE nuclease targeted to *nd4* (bottom left panel), the combination of the mitoTALE nucleases with the mitoTALE nuclease (bottom right panel), or from uninjected cells (top left panel). FIG. 5C is a graph plotting the fold change in deleted mitochondrial genomes with the combined injection of nickase and nucleases, indicating a heteroplasmic shift when the nuclease was included. FIGS. 5D and 5E show the sequences of mtDNAs containing nine different deletions made by the same pair of mitoTALE nickases, confirming the starting and ending positions of the deletions within the mtDNA genome. Target sites for the mitoTALE nickases are boxed. Human mtDNA genome sequence, SEQ ID NOS:20 and 26; deletion 1, SEQ ID NOS:21 and 27; deletion 2, complete deletion and SEQ ID NO:28; deletion 3, SEQ ID NOS:21 and 29; deletion 4, SEQ ID NOS:22 and 30; deletion 5, SEQ ID NOS:23 and 31; deletion 6, SEQ ID NOS:22 and 32; deletion 7, SEQ ID NOS:23 and 32; deletion 8, SEQ ID NOS:24 and 33; deletion 9, SEQ ID NOS:25 and 33.

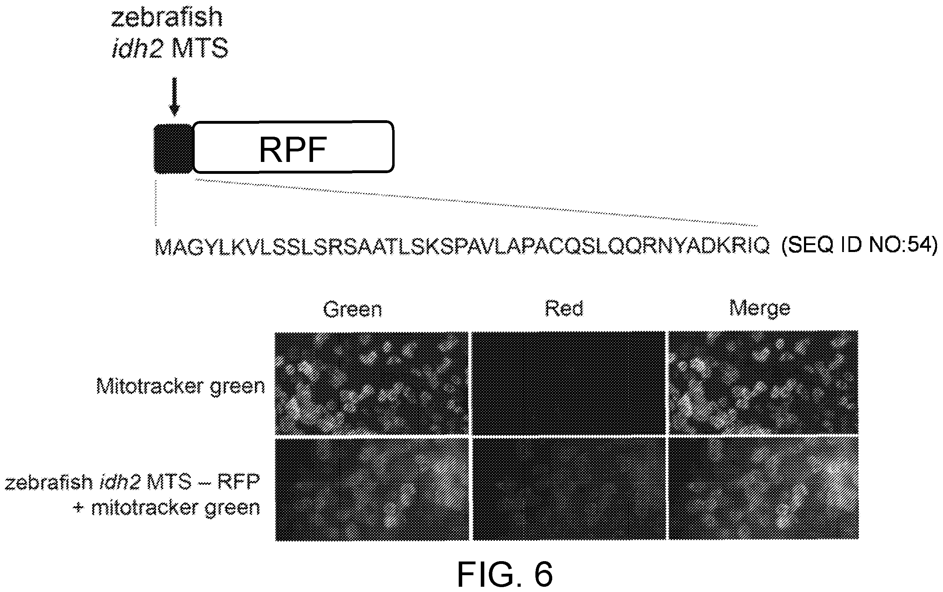

FIG. 6 shows the zebrafish isocitrate dehydrogenase 2 (idh2) mitochondrial targeting sequence (MTS) (top; SEQ ID NO:54), and images of injected zebrafish embryos (bottom). The MTS from GBT268 (zfishbook.com) was characterized using MitoProt (ihg.gsf.de/ihg/mitoprot.html) and fused to RFP for characterization in zebrafish embryos. One-cell embryos were injected with mitotracker green, both with and without the idh2MTS-RFP fusion. After nine hours, embryos were imaged in a Zeiss Lightsheet Z.1.

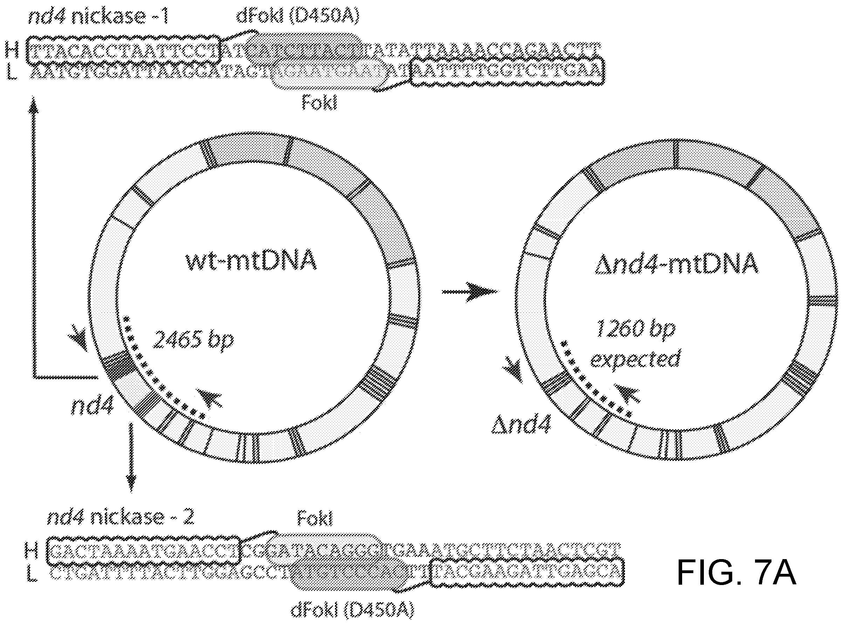

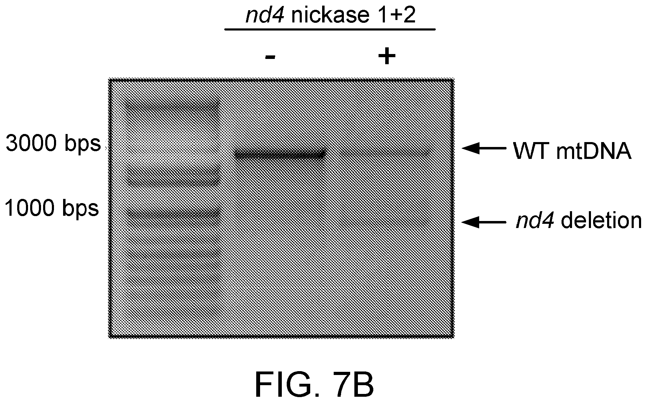

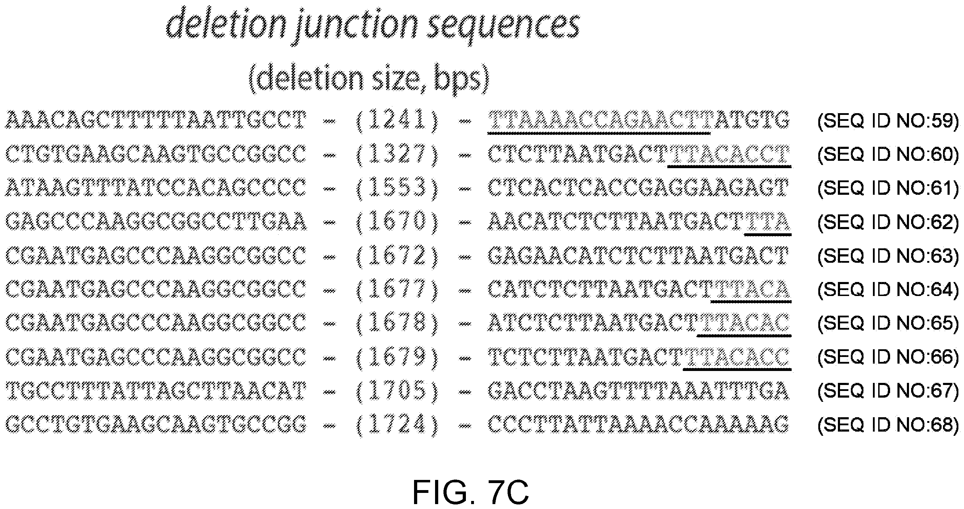

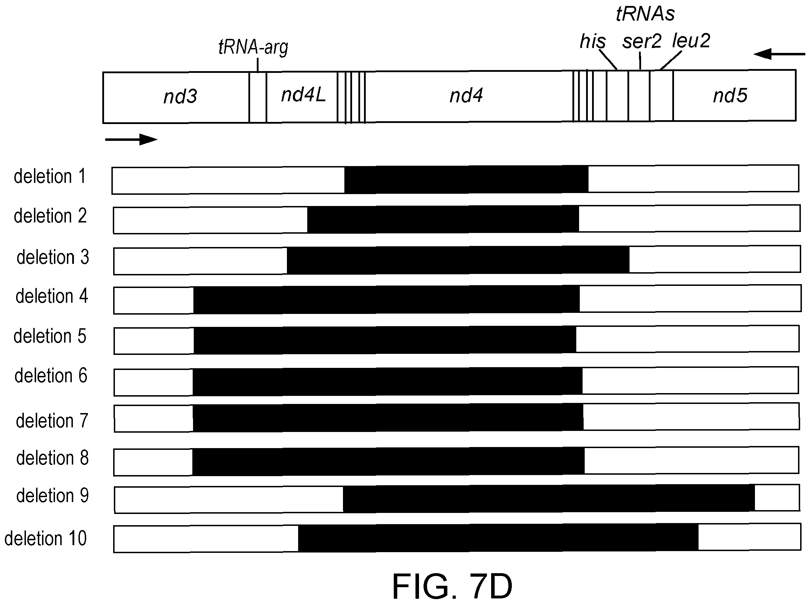

FIGS. 7A-7D demonstrate single gene deletion of nd4 using mitoTALE-nickases. FIG. 7A is a schematic of mtDNA and the target sites for mitoTALE-nickases inside the borders of nd4, with TALE binding to the heavy strand (H) or light strand (L), and *Fok*I activity on either the H strand (bottom) or L strand (top). *dFok*I was catalytically inactive. Deletions were detected using PCR (size approximations indicated by dotted line), with primers positioned outside of both nick sites (arrows). FIG. 7B is an image of a gel indicating deletion of nd4 as detected using PCR on mtDNA from animals injected with nd4 mitoTALE-nickases (right lane). Full-length, non-deleted nd4 mtDNA also was amplified (center lane). FIG. 7C shows the nucleotide sequences of ten deletion junctions, with the size of each deletion indicated in parentheses. Underlined sequences signify that the deletion junction fell in the nd4 nickase-1 binding sites or the spacer. FIG. 7D is a schematic of mtDNA region surrounding nd4, with the locations of deletions shown as black bars.

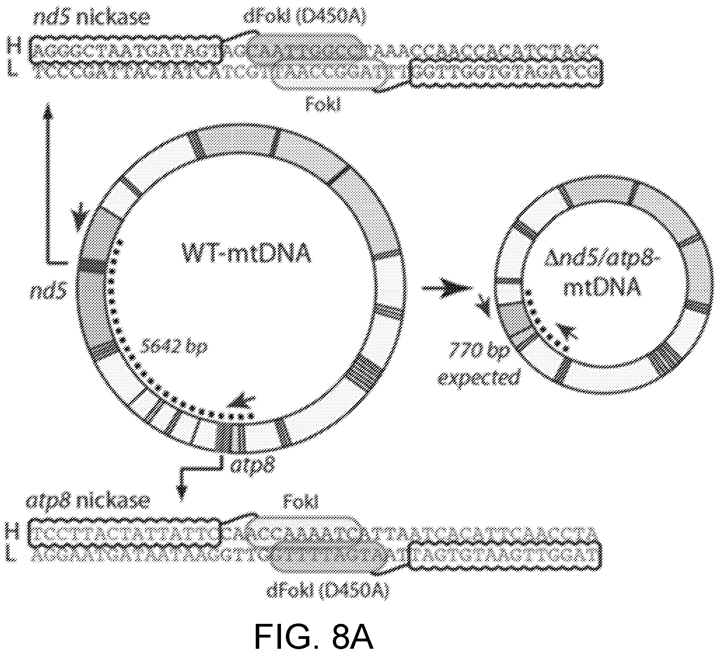

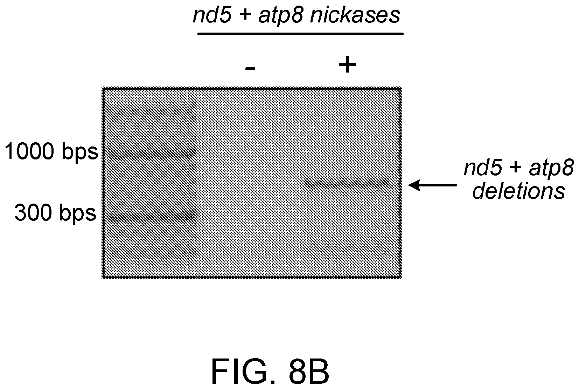

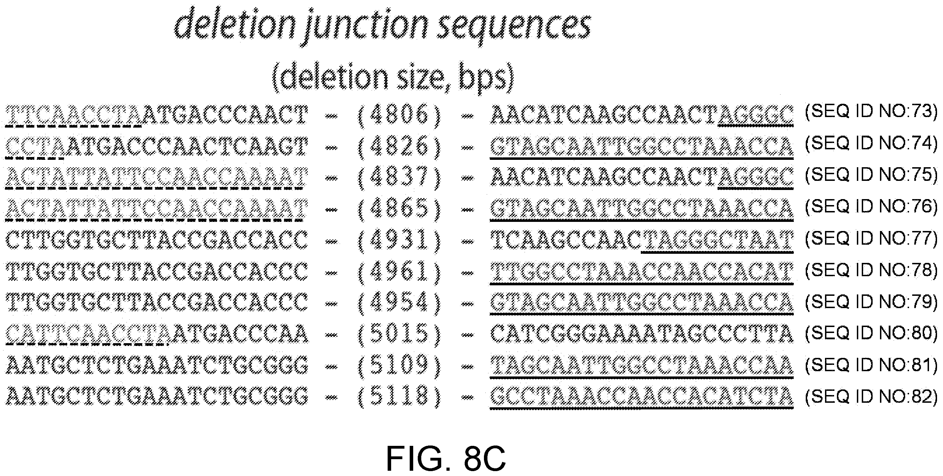

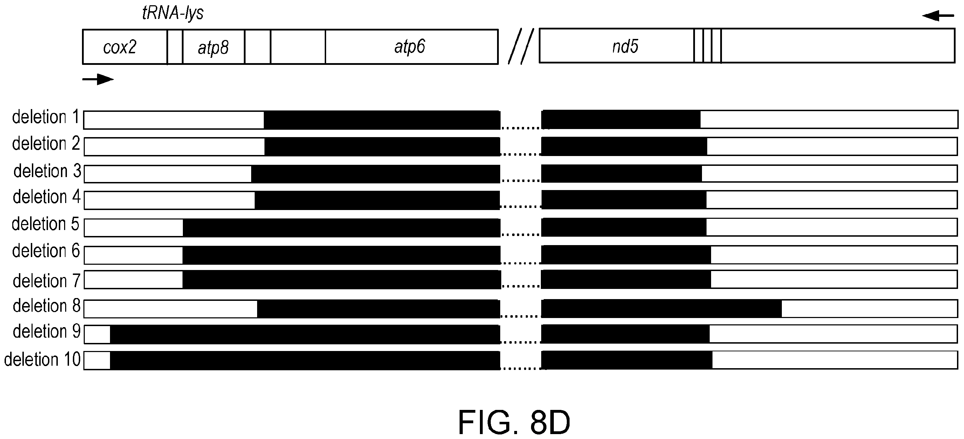

FIGS. 8A-8D demonstrate molecular modeling of a 4977 mtDNA "common deletion" using mitoTALE-nickases. FIG. 8A depicts mitoTALE- nickases targeted to nd5 and atp8, with TALE binding to the heavy strand (H) or light strand (L), and *Fok*I activity on either the H strand (bottom panel) or L strand (top panel). *dFok*I is catalytically inactive. Deletions were detected using PCR, with primers positioned outside of both nick sites (arrows). FIG. 8B is an image of a gel showing deletion of the sequence between the nd5 and atp8 nickases, as detected using bridge-PCR with mtDNA from animals injected with nd5 and atp8 nickases. FIG. 8C shows the nucleotide sequences of ten deletion junctions, with the size each deletion indicated in parentheses. Underlining signifies that the deletion junction fell in the nd5 nickase binding sites or spacer, and dotted underlining signifies that the deletion junction fell in the atp8 nickase binding sites or spacer. FIG. 8D is a schematic of the regions around the atp8 and nd5 nickase binding sites. Black bars indicate deletion locations. All mtDNA between atp6 and nd5 was deleted.

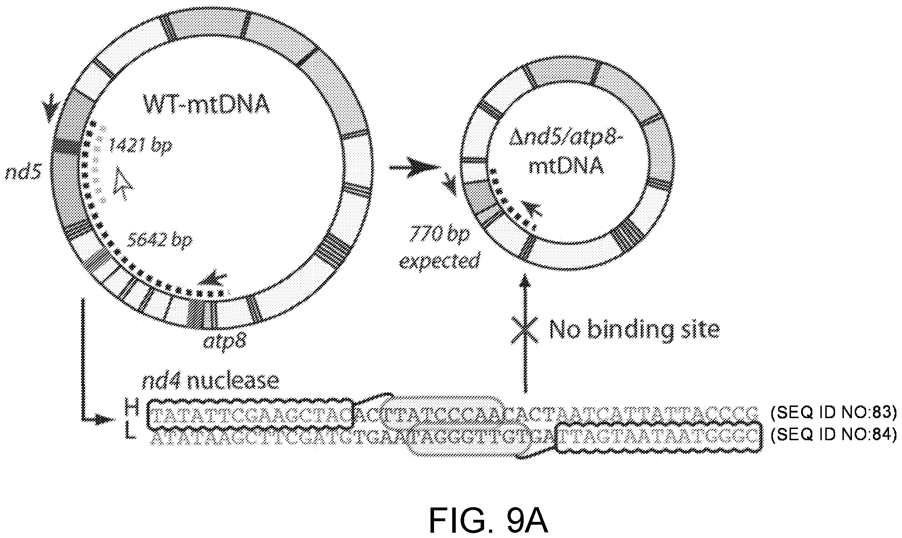

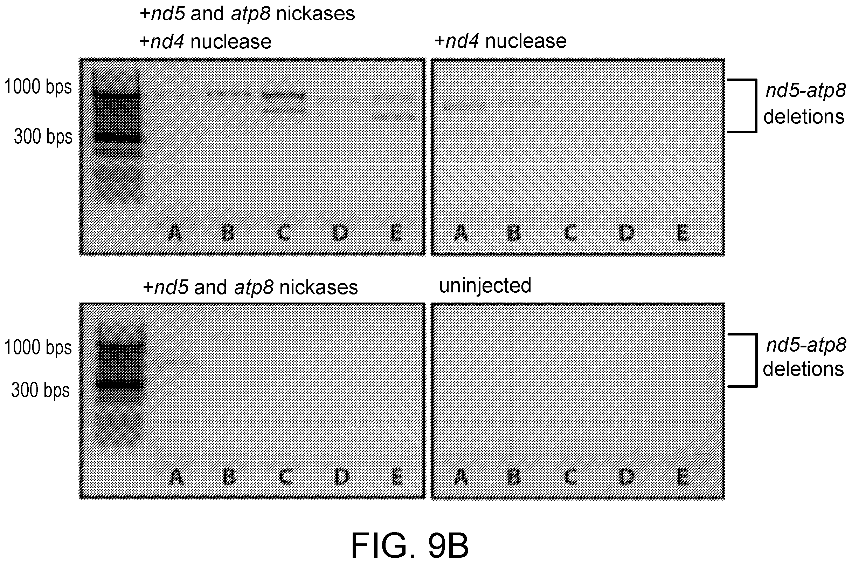

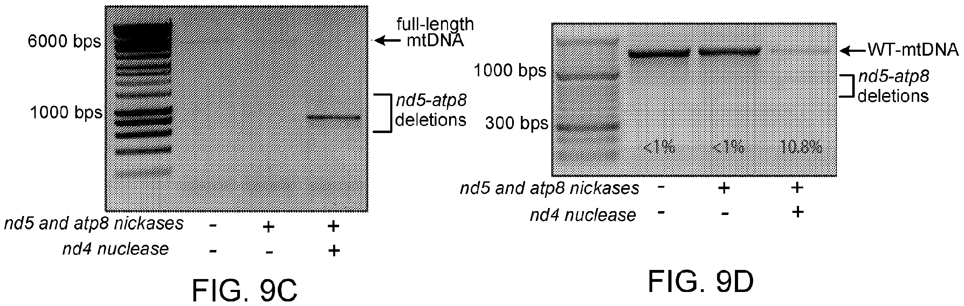

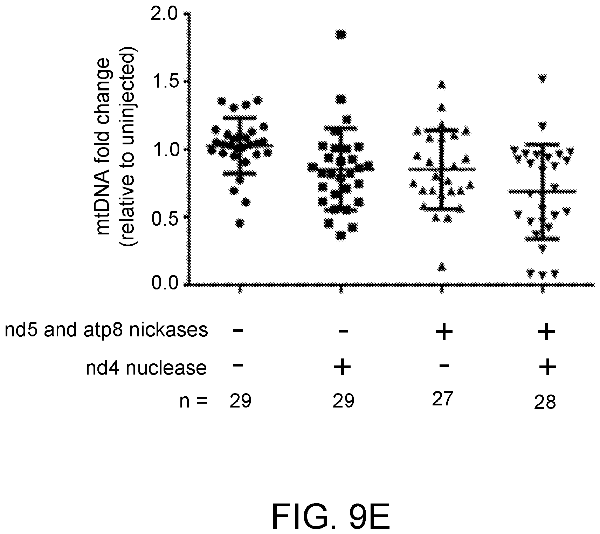

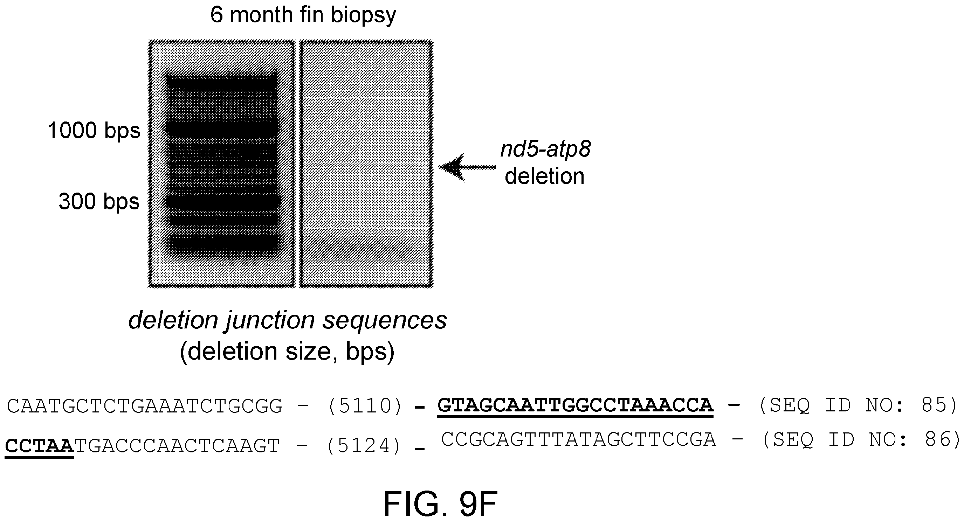

FIGS. 9A-9F demonstrate the heteroplasmic shift of nickase-induced deletions using mitoTALE nucleases. FIG. 9A is a schematic of a mitoTALE-nuclease targeting nd4, designed within the deleted region of Δnd5/atp8-mtDNA, which resulted in targeting to WT-mtDNA but no targeting to Δnd5/atp8-mtDNA due to the lack of the nuclease binding sequence. FIG. 9B contains images of gels from bridge-PCR experiments using mtDNA from zebrafish injected with the atp8 and nd5 mitoTALE-nickases and/or the nd4 mitoTALE-nuclease, as indicated. Bridge-PCR detected deletions between the atp8 and nd5 nick sites from individual embryos (lanes A through E). FIG. 9C is an image of a gel from a long-range PCR assay to detect full-length mtDNA and Δnd5/atp8-mtDNA simultaneously. FIG. 9D is an image of a gel from a three-primer PCR assay to approximate the percent heteroplasmy induced by the atp8 and nd5 nickases alone or with the nd4 nuclease. The primers flanking the nick sites in FIG. 9A detected Δnd5/atp8-mtDNA, while the primer outside nd5 and the primer inside nd5 detected WT-mtDNA. FIG. 9E is a scatter dot plot of mtDNA fold changes in individual fish embryos injected with nickases, nuclease, or both, relative to uninjected animals. Error lines signify standard deviations (bars above and below middle bar) and the mean (middle line). The mtDNA fold change of nickases-alone, nuclease-alone, and both nickases and nuclease-injected animals all were significantly lower compared with uninjected animals (p < 0.05). FIG. 9F contains an image of a gel from a PCR assay (top) and sequences with deletion sizes in parentheses (bottom) from 6-month old adult zebrafish fin biopsy. The underlined sequence indicates that the deletion junction was in the nd5 nickase binding sites or spacer, while the dotted underline indicates the atp8 nickase binding sites or spacer.

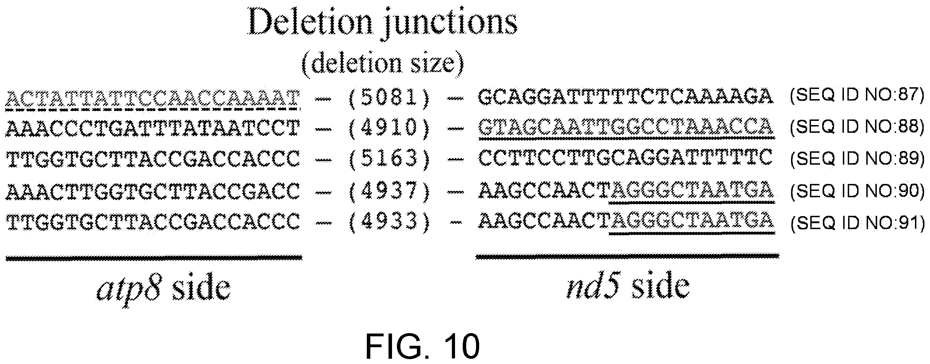

FIG. 10 contains sequence data from embryos injected with both mitoTALE-nickases and mitoTALE nucleases, with deletion sizes indicated in parentheses. The underlined sequence indicates that the deletion junction was in the nd5 nickase binding sites or spacer, while the dotted underline indicates the atp8 nickase binding sites or spacer.

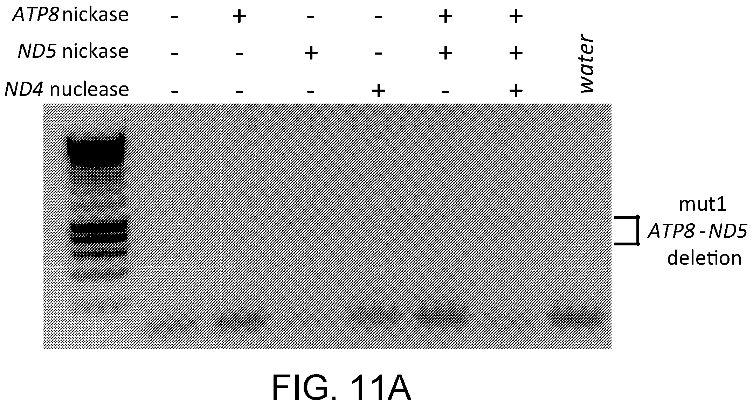

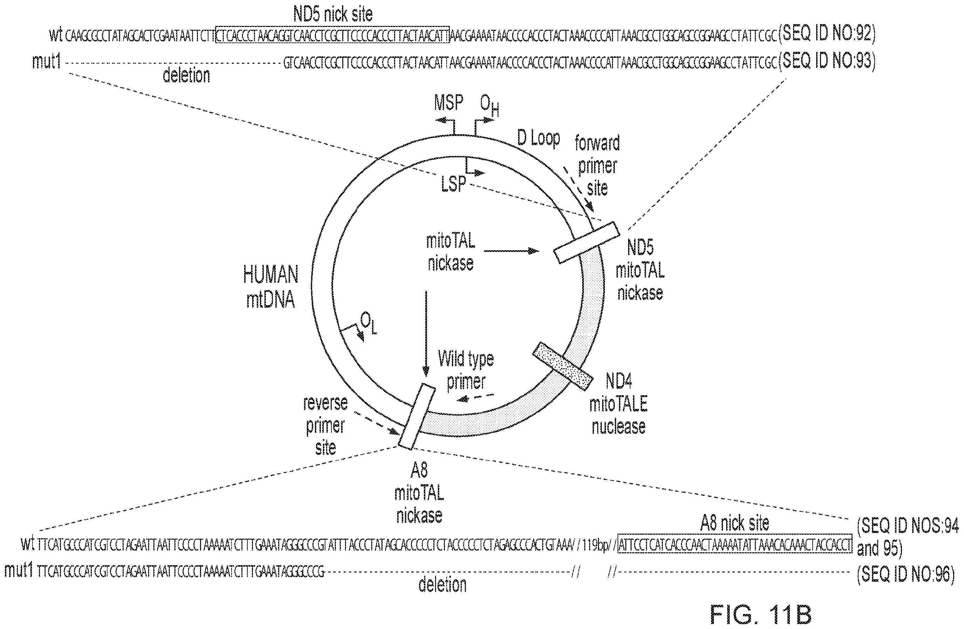

FIGS. 11A and 11B show nickase-induced deletions using mitoTALE nucleases in mammalian cells. FIG. 11A is an image of a gel from a PCR experiments using mtDNA from 293 cells injected with ATP8 and ND5 mitoTALE-nickases and/or the ND4 mitoTALE-nuclease, as indicated. PCR detected deletions between the ATP8 and ND5 nick sites when the ATP8 nickase, ND5 nickase, and ND4 nuclease were cotransfected. FIG. 11B shows the results of sequencing the PCR products, confirm a human mtDNA deletion close to each nick site. The dashes within mutant SEQ ID NOS:93 and 95 shown in FIG. 11B indicate sequences that were deleted. Boxed sequences indicate the nickase sites (including the forward TAL site, reverse TAL site, and 15 bp spacer sequence). A gap of 119 bp is indicated on the A8 side to demonstrate how far the deletion junction is from the A8 nick site (FIG. 11B, bottom sequence).

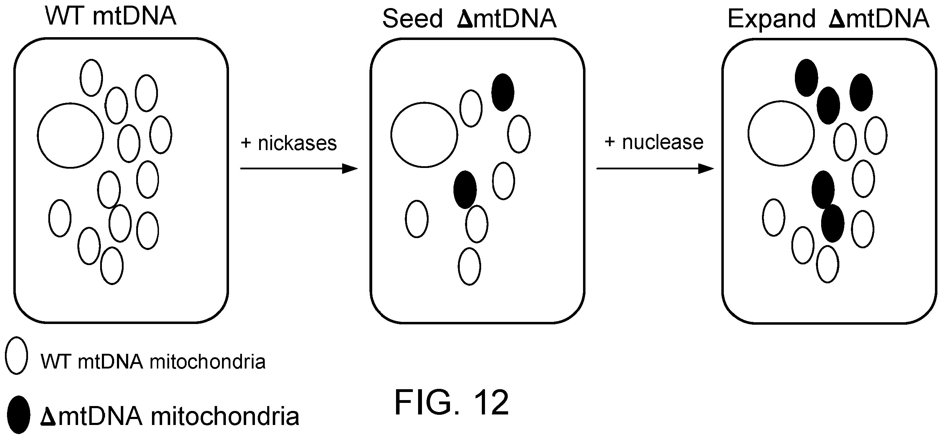

FIG. 12 is a diagram of a two-component mtDNA engineering toolkit using mitoTALE-nickases and nucleases. Targeted deletions are seeded in the mitochondrial network using targeted mitoTALE-nickases, and the resulting designer mitochondrial genomes are separately expanded through heteroplasmic shift from mitoTALE nucleases.

### DETAILED DESCRIPTION

The mitochondrial genome contains double-stranded DNA, but differs from the nuclear genome in that it is circular and much smaller, containing only about 16,500 base pairs that include 37 genes encoding 13 proteins, 22 tRNAs, and 2 rRNAs. Only about three percent of the mitochondrial genome is noncoding DNA. All mitochondrially encoded proteins instruct cells to produce subunits of the enzyme complexes of the oxidative phosphorylation system.

Each cell contains numerous mitochondria, and each mitochondrion contains dozens of copies of the mitochondrial genome. The mitochondrial genome has a mutation rate that is about 100-fold higher than the nuclear genome, which leads to a heterogeneous population of mitochondrial DNA within the same cell (referred to as "heteroplasmy").

Mutations in mtDNA are thought to be associated with numerous clinical disorders. In adults, these include neurological diseases (e.g., migraine, strokes, epilepsy, dementia, myopathy, peripheral neuropathy, diplopia, ataxia, speech disturbances, and sensorineural deafness), gastrointestinal diseases (e.g., constipation, irritable bowel, and dysphagia), cardiac diseases (e.g., heart failure, heart block, and cardiomyopathy), respiratory diseases (e.g., respiratory failure, nocturnal hypoventilation, recurrent aspiration, and pneumonia), endocrine diseases (e.g., diabetes, thyroid disease, parathyroid disease, and ovarian failure), ophthalmological diseases (e.g., optic atrophy, cataract, ophthalmoplegia, and ptosis). In children, disorders thought to be associated with mtDNA mutations include neurological diseases (e.g., epilepsy, myopathy, psychomotor retardation, ataxia, spasticity, dystonia, and sensorineural deafness), gastrointestinal diseases (e.g., vomiting, failure to thrive, and dysphagia), cardiac diseases (e.g., biventricular hypertrophic cardiomyopathy and rhythm abnormalities), respiratory diseases (e.g., central hypoventilation and apnea), hematological diseases (e.g., anemia and pancytopenia), renal diseases (e.g., renal tubular defects), liver diseases (e.g., hepatic failure), endocrine diseases (e.g., diabetes and adrenal failure), and ophthalmological diseases (e.g., optic atrophy). By targeting mtDNA mutations to particular sequences, such clinical conditions can be modeled in experimental systems. For example, zebrafish are a premier teleostean model system, with strong biological and genomic similarities to other vertebrates, and can be useful for studying human biology and disease using *in vivo* genetic and molecular tools, including those described herein.

This document provides materials and methods for manipulating (e.g., modifying, such as through targeted deletions) mtDNA. Studying how changes in mtDNA affect eukaryotic systems can be difficult, because there are limited options for manipulating the mtDNA genome. This document discloses that deletions in the mtDNA genome can be induced, in some embodiments, using a modified TALE nuclease system targeting the mitochondrial genome. The TALE nuclease system can include a heterodimer of a normal and an attenuated form of GoldyTALE nuclease linked to a mitochondrial targeting sequence, referred to herein as an a-mitoTALE nuclease or a mitoTALE nickase, depending on the particular attenuated GoldyTALE nuclease member of the heterodimer. In some embodiments, this site-directed, heterodimeric nuclease system can be targeted to the mitochondrial matrix, where the mtDNA resides submitochondrially. As described in the Examples herein, deletions of about 5 to 6 kb have been generated *in vitro* in human cell culture, and also *in vivo* in *Danio rerio* (zebrafish). The deletions are stable and detectable by PCR, and sequencing revealed that the amplicons match human and zebrafish mitochondrial DNA (respectively), with specific deletions.

The methods provided herein can be used, for example, as research tools for individualized medicine - to make cellular and/or animal avatars of patients with mitochondrial DNA deletions. In addition, this approach can have other applications, especially in mitochondrial research.

The materials and methods provided herein can be used to introduce genetic changes precisely at endonuclease cut sites *in vitro.* For example, in some embodiments, the methods can be used to introduce deletions into particular mitochondrial genes, in order to mimic clinical disorders characterized by mutations in those genes. The genetic changes can be induced using chimeric DNA nickase polypeptides that include (a) a mitochondrial target sequence directing the chimeric molecules to mitochondria, (b) a region designed to target the molecules to specific DNA sequences within the mitochondria, and (c) a nuclease that generates a single strand cut in the mitochondrial DNA at or near the selected target sequence.

Suitable mitochondrial target sequences include, for example, the mitochondrial targeting sequence associated with the isocitrate dehydrogenase 2 protein, as well as the human COX8A MTS, human SOD2 MTS. Other useful targeting sequences include those described elsewhere *(see,* for example, Claros and Vincens, Eur. J. Biochem. 241:779-786, 1996).

In some embodiments, the region of the chimeric nuclease polypeptide that targets the molecule to a particular DNA sequence can include a sequence from a TALE molecule. Alternatively, the region targeting the chimeric DNA nuclease to a selected sequence can be from a zinc finger protein (e.g., a zinc finger DNA binding domain derived from the mouse transcription factor Zif268; Porteus and Baltimore, Science 300:763, 2003) or a meganuclease (e.g., a wild type or variant homing endonuclease, such as a meganuclease belonging to the dodecapeptide family (LAGLIDADG; SEQ ID NO:34; *see,* WO 2004/067736).

In some embodiments, DNA targeting sequences derived from TALEs can be particularly useful. TALEs are polypeptides of plant pathogenic bacteria that are injected by the pathogen into the plant cell, where they travel to the nucleus and function as transcription factors to turn on specific plant genes *(see,* e.g., Gu et al., Nature 435:1122, 2005; Yang et al., Proc. Natl. Acad. Sci. USA 103:10503, 2006; Kay et al., Science 318:648, 2007; Sugio et al., Proc. Natl. Acad. Sci. USA 104:10720, 2007; and Römer et al., Science 318:645, 2007). Specificity depends on an effector-variable number of imperfect, typically 34 amino acid repeats (Schornack et al., J. Plant Physiol. 163:256, 2006). Polymorphisms are present primarily at repeat positions 12 and 13, which are referred to as the repeat variable-diresidue (RVD). Interestingly, the RVDs of TALEs correspond to the nucleotides in their target sites in a direct, linear fashion, one RVD to one nucleotide, with some degeneracy and no apparent context dependence. Since the primary amino acid sequence of a TALE dictates the nucleotide sequence to which it binds, target sites can be predicted for TALEs, and TALEs also can be engineered and generated for the purpose of binding to particular nucleotide sequences.

Further, by linking a TALE to an endonuclease, a sequence-specific TALE nuclease can be designed and generated to recognize a preselected target nucleotide sequence present in a cell. In some cases, a target nucleotide sequence can be scanned for nuclease recognition sites, and a particular nuclease can be selected based on the target sequence. In some cases, a TALE nuclease can be engineered to target a particular cellular (e.g., mtDNA) sequence. A nucleotide sequence encoding the desired TALE nuclease can be inserted into any suitable expression vector, and can be operably linked to one or more promoters or other expression control sequences, as described further below.

Examples and further descriptions of TALE nucleases can be found, for example, in U.S. Patent No. 8,586,363. In some cases, a TALE nuclease can have truncations at the N- and/or C-terminal regions of the TAL portion of the polypeptide, such that it has a shortened scaffold as compared to a wild type TAL polypeptide. An exemplary TALE nuclease with a modified scaffold is the +63 TALE nuclease described, for example, in PCT Publication No. WO 2013/191769. It is to be noted that the TAL portion also can include one or more additional variations (e.g., substitutions, deletions, or additions) in combination with such N- and C-terminal scaffold truncations. For example, a TALE nuclease can have N- and C-terminal truncations of the TAL portion in combination with one or more amino acid substitutions (e.g., within the scaffold and/or within the repeat region).

In general, for mitoTALE nuclease polypeptides, a TALE sequence can be fused to a mitochondrial targeting sequence, as described above, and also to a nuclease or a portion of a nuclease - such as a nonspecific cleavage domain from a type II restriction endonuclease such as *Fok*I (Kim et al., Proc. Natl. Acad. Sci. USA, 93:1156-1160, 1996). Other useful endonucleases may include, for example, *Hha*I, *Hind*III, *Not*I, *BbvC*I, *EcoR*I, *BglI,* and *Alw*I. In some cases, the fact that some endonucleases (e.g., *Fok*I) function as dimers (Bitinaite et al., Proc. Natl Acad. Sci. USA 95:10570-10575, 1998) can be capitalized upon to enhance the target specificity of the TALE. For example, in some cases, separate *Fok*I monomers can be fused to TALE sequences that recognize different DNA target sequences, and only when the two recognition sites are in close proximity do the inactive monomers come together to create a functional enzyme. By requiring DNA binding to activate the nuclease, a highly site-specific restriction enzyme can be created. In some embodiments, however, monomeric TALE nucleases can be constructed, such that single TALEs are fused to a nuclease that does not require dimerization to function. One such nuclease, is a single-chain variant of *Fok*I in which the two monomers are expressed as a single polypeptide *(see,* Minczuk et al., Nucleic Acids Res. 36:3926-3938, 2008).

It is to be noted that to generate a nuclease that cleaves double strand DNA at a lower efficiency, or that cleaves single strand DNA to generate a nick rather than a double strand break, a *Fok*I sequence can be mutated to alter its activity. For example, the aspartic acid residue at position 483 or position 450 of a *Fok*I endonuclease can be replaced with an alanine residue, resulting in a nuclease that nicks rather than fully cleaves double stranded DNA, or that cleaves double stranded DNA but with a lower efficiency than non-mutagenized *Fok*I. In some embodiments, an a-mitoTALE nuclease or mitoTALE nickase can include a *Fok*I heterodimer in which one monomer contains the wild type *Fok*I amino acid sequence, while the other monomer contains a *Fok*I amino acid sequence with a D483A or D450A mutation.

In some embodiments, a chimeric DNA nickase can include a mitochondrial targeting sequence fused to a Clustered Regularly Interspersed Short Palindromic Repeats (CRISPR) associated system (Cas) polypeptide. In its native context, the CRISPR/Cas system provides bacteria and archaea with immunity to invading foreign nucleic acids (Jinek et al., Science 337:816-821, 2012). The CRISPR/Cas system is functionally analogous to eukaryotic RNA interference, using RNA base pairing to direct DNA or RNA cleavage. This process relies on (a) small RNAs that base-pair with sequences carried by invading nucleic acid, and (b) a specialized class of Cas endonucleases that cleave nucleic acids complementary to the small RNA. In some embodiments, a Cas9 endonuclease (e.g., a *Streptococcus pyogenes* Cas9 endonuclease) can be used. The CRISPR/Cas system can be reprogrammed to create targeted double-strand DNA breaks in higher-eukaryotic genomes, including animal and plant cells (Mali et al., Science 339:823-826, 2013; and Li et al., Nature Biotechnol. 31(8):688-691, 2013). Further, by modifying specific amino acids in the Cas protein that are responsible for DNA cleavage, the CRISPR/Cas system can function as a DNA nickase (Jinek et al., *supra).* For example, Cas9 nuclease proteins having a D10A or H840A mutation can have nickase activity.

Directing the Cas9 protein to a particular sequence requires crRNA and tracrRNA sequences that aid in directing the Cas/RNA complex to target DNA sequence (Makarova et al., Nat. Rev. Microbiol., 9(6):467-477, 2011). The modification of a single targeting RNA can be sufficient to alter the nucleotide target of a Cas protein. In some cases, crRNA and tracrRNA can be engineered as a single cr/tracrRNA hybrid to direct Cas activity (also referred to as a "guide RNA" or gRNA). Thus, introduction of a chimeric Cas nickase into a cell with one or more crRNA and tracrRNA sequences (or one or more gRNA sequences) that are targeted to one or more mtDNA sequences, where the chimeric Cas nickase includes a mitochondrial targeting sequence and the gRNA or crRNA and tracrRNA are linked to a mitochondrial RNA targeting sequence *(see,* e.g., Sieber et al., Nucl. Acids Res. doi:10.1093/nar/gkr380, 2011) can direct the gRNA/crRNA and tracrRNA and the Cas nickase to the selected mtDNA sequence.

Methods for altering the mtDNA of a eukaryotic cell (e.g., a cell in culture or a cell within a eukaryotic organism, including a non-human vertebrate such as a zebrafish, a mouse, a rat, a rabbit, a sheep, a pig, or a dog) can include introducing one or more (e.g., one, two, three, four, five, six, or more than six) recombinant DNA nucleases and/or nickases (e.g., a-mitoTALE nucleases and/or mitoTALE nickases) into the cell, either by introducing one or more nuclease and/or nickase polypeptides or by introducing nucleic acid encoding one or more nuclease and/or nickase polypeptides. For example, an a-mitoTALE nuclease can be introduced into a cell as a nucleic acid vector encoding the chimeric polypeptide, or as a polypeptide *per se.* Any suitable a-mitoTALE nuclease or mitoTALE nickase can be used in the methods described herein. In some embodiments, for example, an a-mitoTALE nuclease or mitoTALE nickase having a TALE scaffold based on or including SEQ ID NOS:1 and 2 (FIG. 1, bottom panel) can be used. In some cases, more than one (e.g., two, three, four, five, six, or more than six) a-mitoTALE nucleases or mitoTALE nickases can be introduced into a cell or an organism, with each a-mitoTALE nuclease and nickase targeting a different sequence. Such methods can lead to multiple nicks in the mitochondrial DNA within the cell or the organism, and in some cases (e.g., as in the Examples below) can lead to a deletion of several kb of genomic mtDNA sequence.

In some embodiments, one or more a-mitoTALE nucleases or mitoTALE nickases can be introduced into a cell in combination with a mitoTALE nuclease that does not have attenuated activity. As described in Example 5 herein, methods that include such a combination of molecules can result in an increased level of mitochondrial DNA deletion, and increased heteroplasmy as compared to the level of heteroplasmy achieved using a-mitoTALE nucleases or mitoTALE nickases alone. Thus, a method can include introducing into a cell one or more nucleic acid vectors that encode an a-mitoTALE nuclease or a mitoTALE nickase, in combination with a nucleic acid vector encoding a mitoTALE nuclease, or the a-mitoTALE nuclease, mitoTALE nickase, and mitoTALE nuclease polypeptides may be introduced into the cell. In some embodiments, the mitoTALE nuclease may be targeted to a sequence within the region of mtDNA that is targeted by the a-mitoTALE nucleases or mitoTALE nickases for deletion.

Isolated chimeric DNA nuclease and nickase nucleic acids and polypeptides that are targeted to mtDNA also are provided herein. The term "polypeptide" as used herein refers to a compound of two or more subunit amino acids regardless of post-translational modification (e.g., phosphorylation or glycosylation). The subunits may be linked by peptide bonds or other bonds such as, for example, ester or ether bonds. The term "amino acid" refers to either natural and/or unnatural or synthetic amino acids, including D/L optical isomers.

By "isolated" or "purified" with respect to a polypeptide it is meant that the polypeptide is separated to some extent from the cellular components with which it is normally found in nature (e.g., other polypeptides, lipids, carbohydrates, and nucleic acids). A purified polypeptide can yield a single major band on a non-reducing polyacrylamide gel. A purified polypeptide can be at least about 75% pure (e.g., at least 80%, 85%, 90%, 95%, 97%, 98%, 99%, or 100% pure). Purified polypeptides can be obtained by, for example, extraction from a natural source, by chemical synthesis, or by recombinant production in a host cell or transgenic plant, and can be purified using, for example, affinity chromatography, immunoprecipitation, size exclusion chromatography, and ion exchange chromatography. The extent of purification can be measured using any appropriate method, including, without limitation, column chromatography, polyacrylamide gel electrophoresis, or high-performance liquid chromatography.

The terms "nucleic acid" and "polynucleotide" are used interchangeably, and refer to both RNA and DNA, including cDNA, genomic DNA, synthetic (e.g., chemically synthesized) DNA, and DNA (or RNA) containing nucleic acid analogs. Polynucleotides can have any three-dimensional structure. A nucleic acid can be double-stranded or single-stranded (i.e., a sense strand or an antisense single strand). Non-limiting examples of polynucleotides include genes, gene fragments, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers, as well as nucleic acid analogs.

As used herein, "isolated," when in reference to a nucleic acid, refers to a nucleic acid that is separated from other nucleic acids that are present in a genome, e.g., a plant genome, including nucleic acids that normally flank one or both sides of the nucleic acid in the genome. The term "isolated" as used herein with respect to nucleic acids also includes any non-naturally-occurring sequence, since such non-naturally-occurring sequences are not found in nature and do not have immediately contiguous sequences in a naturally-occurring genome.

An isolated nucleic acid can be, for example, a DNA molecule, provided one of the nucleic acid sequences normally found immediately flanking that DNA molecule in a naturally-occurring genome is removed or absent. Thus, an isolated nucleic acid includes, without limitation, a DNA molecule that exists as a separate molecule (e.g., a chemically synthesized nucleic acid, or a cDNA or genomic DNA fragment produced by PCR or restriction endonuclease treatment) independent of other sequences, as well as DNA that is incorporated into a vector, an autonomously replicating plasmid, a virus (e.g., a pararetrovirus, a retrovirus, lentivirus, adenovirus, or herpes virus), or the genomic DNA of a prokaryote or eukaryote. In addition, an isolated nucleic acid can include a recombinant nucleic acid such as a DNA molecule that is part of a hybrid or fusion nucleic acid. A nucleic acid existing among hundreds to millions of other nucleic acids within, for example, cDNA libraries or genomic libraries, or gel slices containing a genomic DNA restriction digest, is not to be considered an isolated nucleic acid.

A nucleic acid can be made by, for example, chemical synthesis or polymerase chain reaction (PCR). PCR refers to a procedure or technique in which target nucleic acids are amplified. PCR can be used to amplify specific sequences from DNA as well as RNA, including sequences from total genomic DNA or total cellular RNA. Various PCR methods are described, for example, in PCR Primer: A Laboratory Manual, Dieffenbach and Dveksler, eds., Cold Spring Harbor Laboratory Press, 1995. Generally, sequence information from the ends of the region of interest or beyond is employed to design oligonucleotide primers that are identical or similar in sequence to opposite strands of the template to be amplified. Various PCR strategies also are available by which site-specific nucleotide sequence modifications can be introduced into a template nucleic acid.

Isolated nucleic acids also can be obtained by mutagenesis. For example, a donor nucleic acid sequence can be mutated using standard techniques, including oligonucleotide-directed mutagenesis and site-directed mutagenesis through PCR. See, Short Protocols in Molecular Biology, Chapter 8, Green Publishing Associates and John Wiley & Sons, Ausubel et al. (Ed.), 1992.

This document also provides nucleic acid vectors containing nucleotide sequences that encode one or more chimeric DNA nuclease or nickase (e.g., a-mitoTALE nuclease) molecules. A "vector" is a replicon, such as a plasmid, phage, or cosmid, into which another DNA segment may be inserted so as to bring about the replication of the inserted segment. Generally, a vector is capable of replication when associated with the proper control elements. Suitable vector backbones include, for example, those routinely used in the art such as plasmids, viruses, artificial chromosomes, BACs, YACs, or PACs. The term "vector" includes cloning and expression vectors, as well as viral vectors and integrating vectors. An "expression vector" is a vector that includes one or more expression control sequences, and an "expression control sequence" is a DNA sequence that controls and regulates the transcription and/or translation of another DNA sequence. Suitable expression vectors include, without limitation, plasmids and viral vectors derived from, for example, bacteriophage, baculoviruses, tobacco mosaic virus, herpes viruses, cytomegalovirus, retroviruses, vaccinia viruses, adenoviruses, and adeno-associated viruses. Numerous vectors and expression systems are commercially available from such corporations as Novagen (Madison, WI), Clontech (Palo Alto, CA), Stratagene (La Jolla, CA), and Invitrogen/Life Technologies (Carlsbad, CA).

The nucleic acids provided herein can include a "regulatory region" (also referred to as a "control element" or "expression control sequence"), which is a nucleotide sequence that influences transcription or translation initiation and rate, and/or stability or mobility of the transcript or polypeptide product. Regulatory regions include, without limitation, promoter sequences, enhancer sequences, response elements, protein recognition sites, inducible elements, promoter control elements, protein binding sequences, 5' and 3' untranslated regions (UTRs), transcriptional start sites, termination sequences, polyadenylation sequences, introns, and other regulatory regions that can reside within coding sequences, such as secretory signals, mitochondrial targeting sequences, and protease cleavage sites.

As used herein, "operably linked" means incorporated into a genetic construct so that expression control sequences effectively control expression of a coding sequence of interest. A coding sequence is "operably linked" and "under the control" of expression control sequences in a cell when RNA polymerase is able to transcribe the coding sequence into RNA, which if an mRNA, then can be translated into the protein encoded by the coding sequence. Thus, a regulatory region can modulate, e.g., regulate, facilitate or drive, transcription in the plant cell, plant, or plant tissue in which it is desired to express a modified target nucleic acid.

A promoter is an expression control sequence composed of a region of a DNA molecule, typically within 100 nucleotides upstream of the point at which transcription starts (generally near the initiation site for RNA polymerase II). Promoters are involved in recognition and binding of RNA polymerase and other proteins to initiate and modulate transcription. To bring a coding sequence under the control of a promoter, it typically is necessary to position the translation initiation site of the translational reading frame of the polypeptide between one and about fifty nucleotides downstream of the promoter. A promoter can, however, be positioned as much as about 5,000 nucleotides upstream of the translation start site, or about 2,000 nucleotides upstream of the transcription start site. A promoter typically comprises at least a core (basal) promoter. A promoter also may include at least one control element such as an upstream element. Such elements include upstream activation regions and, optionally, other DNA sequences that affect transcription of a polynucleotide such as a synthetic upstream element.

The choice of promoters to be included depends upon several factors, including, but not limited to, efficiency, selectability, inducibility, desired expression level, and cell or tissue specificity. For example, tissue-, organ- and cell-specific promoters that confer transcription only or predominantly in a particular tissue, organ, and cell type, respectively, can be used. Alternatively, constitutive promoters can promote transcription of an operably linked nucleic acid in most or all tissues, throughout development. Other classes of promoters include, without limitation, inducible promoters, such as promoters that confer transcription in response to external stimuli (e.g., chemical agents, developmental stimuli, or environmental stimuli).

The vectors provided herein also can include, for example, origins of replication, and/or scaffold attachment regions (SARs). In addition, an expression vector can include a tag sequence designed to facilitate manipulation or detection (e.g., purification or localization) of the expressed polypeptide. Tag sequences, such as green fluorescent protein (GFP), glutathione S-transferase (GST), polyhistidine, c-myc, hemagglutinin, or Flag™ tag (Kodak, New Haven, CT) sequences typically are expressed as a fusion with the encoded polypeptide. Such tags can be inserted anywhere within the polypeptide, including at either the carboxyl or amino terminus.

The terms "vector" or "vectors" refer to a nucleic acid molecule capable of transporting another nucleic acid to which it has been linked. A "vector" in the present document includes, but is not limited to, a viral vector, a plasmid, a RNA vector or a linear or circular DNA or RNA molecule which may consists of a chromosomal, non-chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Recombinant nucleic acid constructs can include a polynucleotide sequence inserted into a vector suitable for transformation of cells (e.g., plant cells or animal cells). Recombinant vectors can be made using, for example, standard recombinant DNA techniques *(see,* e.g., Sambrook et al. (1989) Molecular Cloning: A Laboratory Manual, 2nd ed., Cold Spring Harbor Laboratory, Cold Spring Harbor, NY).

Vectors can be introduced into eukaryotic cells or non-human organisms (e.g., plants, including monocots and dicots, or animals, including fish, rodents, sheep, pigs, cows, dogs, cats, and primates) by any of a variety of methods (e.g., injection, direct uptake, projectile bombardment, liposomes, or electroporation). As described in the Examples herein, for example, DNA or RNA encoding one or more (e.g., one, two, three, four, five, six, or more than six) mitoTALE nucleases, a-mitoTALE nucleases, or mitoTALE nickases, or a combination of one or more nucleases and one or more nickases (e.g., two mitoTALE nickases and an a-mitoTALE nuclease or a mitoTALE nuclease), can be transformed into a cell or microinjected into an organism such as a zebrafish (e.g., a zebrafish embryo). The cell (or progeny thereof) or organism can be assessed using, for example, PCR and sequencing techniques to determine whether a deletion has been generated at or near the targeted sequence(s), and to assess the sizes of any such deletions. In some examples, a transformed organism (e.g., a non-human embryo) can be allowed to develop, and the resulting organism can be assessed to determine whether the deletions have been maintained.

The invention will be further described in the following examples, which do not limit the scope of the invention described in the claims.

### EXAMPLES

### Example 1 - Heterodimeric a-mitoTALE nuclease protein structure and sequence

The protein structure for an a-mitoTALE nuclease is shown in the top panel of FIG. 1. The mitoTALE nuclease system with heterodimeric subunits (a-mitoTALE nuclease) includes a zebrafish isocitrate dehydrogenase 2 (*idh2*) mitochondrial targeting sequence fused to the GoldyTALE backbone (Bedell et al., Nature 491:114-118, 2012). The protein sequence of the a-mitoTALE nuclease is shown in the bottom panel of FIG. 1. The sequence of the zebrafish *idh2* mitochondrial targeting sequence is in bold, the GoldyTALE backbone sequence is italicized, and sequence of the *Fok*I domain is underlined. To make an attenuated *Fok*I, a single aspartate to alanine point mutation was made to *Fok*I at position 483 (circled in FIG. 1). The protein is completed by a series of tandem repeats containing RVDs that are inserted into the GoldyTALE sequence; the RVDs within the tandem repeats direct the construct to a specific genomic locus. The sequences of the RVD-containing repeats are not shown, but their position is indicated by a black box in both panels of FIG. 1. Methods for designing TALE molecules targeted to selected DNA sequences are known in the art. *See,* for example U.S. Patent No. 8,586,363.

### Example 2 - mtDNA deletions in zebrafish made by a-mitoTALE nucleases

a-mitoTALE nucleases were generated to target the *nd5* (NADH dehydrogenase subunit 5) and *coII* (cytochrome c oxidase subunit II) genes in the zebrafish mitochondrial genome (FIG. 2A, black bars). The expected deletion size was about 6 kb. Primers were made to face toward the expected deletion (dotted arrows), such that a PCR product would only be detected if a deletion had occurred. Indeed, after injection of the a-mitoTALE nucleases into one-cell zebrafish embryos and isolation of DNA after four days of embryonic development, PCR confirmed the presence of mtDNA deletions. Uninjected fish (FIG. 2B, center lane) did not show a band of the expected size, while the a-mitoTALE nuclease-injected fish did (FIG. 2B, right lane, arrow). To confirm that the PCR products were mtDNA, several bands were sequenced. This revealed that the same a-mitoTALE nuclease pair, when injected into multiple fish, resulted in a range of deletions. Five examples are shown in FIG. 2C, with the original a-mitoTALE nuclease binding sites and spacers boxed. Colons indicate deleted nucleotides. The deletion sizes were roughly 6 kb, but the sizes varied by up to several hundred nucleotides in difference.

### Example 3 - mtDNA deletions in human cell culture made by a-mitoTALE nucleases

a-mitoTALE nucleases also were generated to target the mitochondrial ND5 (NADH dehydrogenase subunit 5) and A8 (ATPase subunit 8) genes in human 293T cells (FIG. 3A, black bars). The expected deletion size was about 5 kb. Primers were made facing toward the expected deletion (dotted arrows), such that a PCR product would only be detected if a deletion had occurred. PCR confirmed that cells transfected with the a-mitoTALE nucleases and incubated at 37°C for 2 to 7 days before DNA isolation exhibited mtDNA deletions, as evidenced by a band of the expected size (FIG. 3B, right lane, arrow; band was observed after 2 days of incubation). In contrast, cells transfected with GFP did not exhibit such a band. To confirm that the PCR products were mtDNA, several bands were sequenced. These studies revealed that a range of deletion sizes were induced with the same a-mitoTALE nuclease pair. Five examples are shown in FIG. 3C, with the original a-mitoTALE nuclease binding site and spacer boxed. Colons indicate deleted nucleotides. A range of deletion sizes was observed, differing by up to several hundred nucleotides.

### Example 4 - Heterodimeric mitoTALE nickase protein structure and sequence

A more "traditional" mitoTALE nickase was generated by replacing the aspartic acid at position 450 of the *Fok*I sequence with an alanine. A diagram of the heterodimeric mitoTALE nickase is shown in FIG. 4. The protein includes a zebrafish *idh2* mitochondrial targeting sequence (hatched box in structure, bold text in sequence), a GoldyTALE backbone (dotted bFox, italics in sequence) with sequences containing repeat variable diresidues (RVDs) inserted (black boxes in structure and text), and a *Fok*I/d*Fok*I dimer sequence (gray box in structure, underlined text in sequence). The boxed aspartic acid at position 450 of the *Fok*I sequence indicates the position of the Asp to Ala mutation that renders the *Fok*I endonuclease domain catalytically inactive.

### Example 5 - Increased heteroplasmy induced by simultaneous injection of mitoTALE nickases and a mitoTALE nuclease

Experiments were conducted to determine the effect of using mitoTALE nickases in combination with a mitoTALE endonuclease targeted to the mitochondrial sequence to be deleted. As indicated in FIG. 5C, these studies revealed that simultaneous delivery of a mitoTALE nuclease can increase the number of deleted mtDNA genomes as compared to mitoTALE nickases alone. mitoTALE nickases targeted to the *nd5* and *atp8* genes (FIG. 5A, black bars) and a mitoTALE nuclease targeted to the nd4 gene (FIG. 5A, hatched bar) were injected into zebrafish separately or in combination. Short range PCR using DNA isolated from injected zebrafish revealed a higher proportion of deleted genomes when the combination of mitoTALE nickases and mitoTALE nuclease was used (FIG. 5B, arrow).

To quantify this change, real-time PCR was run to detect deletions, and products were normalized to a nuclear gene (FIG. 5C). Co-delivery of the mitoTALE nickases and the mitoTALE nuclease resulted in about a 20-fold increase in the number of deleted mtDNA genomes, as compared with delivery of mitoTALE nickases alone. Thus, a heteroplasmic shift occurred when the nuclease was included.

In addition, the mtDNAs were sequenced to confirm the starting and ending positions of the deletions within the mtDNA genome. Nine different deletions were generated made by the pair of *nd5* and *atp8* mitoTALE nickases in combination with the *nd4* nuclease (FIGS. 5D and 5E).

### Example 6 - Designer mitochondrial genomes using two-component engineering Methods

*MitoTALE-nickase and -nuclease assembly:* The mitochondrial targeting sequence of idh2 (FIG. 6) was cloned into pT3TS29, and was subsequently fused to GoldyTALE nuclease with the nuclear localization sequence removed (Bedell et al., *supra).* To make the mitoTALE-nickase, a D450A substitution in the *Fok*I domain of GoldyTALE nuclease was made (Waugh and Sauer, Proc. Natl. Acad. Sci. USA 90:9596-9600, 1993). To target a specific sequence in the genome, RVDs were cloned into the GoldyTALE backbone using the FusX assembly system (Ma et al., Hum. Gene Ther. 2016, doi:10.1089/hum.2015.172). The RVDs used were HD=C, NN=G, NI=A, NG=T. The sites targeted by nickases and nucleases were as follows: zebrafish nd5 forward mitoTALE-nickase, 5'-AGGGCTAATGATAGT-3' (SEQ ID NO:35); zebrafish nd5 reverse mitoTALE-nickase, 5'-GCTAGATGTGGTTGG-3' (SEQ ID NO:36); zebrafish atp8 forward mitoTALE-nickase, 5'-TCCTTACTATTATTC-3' (SEQ ID NO:37); zebrafish atp8 reverse mitoTALE-nickase, 5'-TAGGTTGAATGTGAT-3' (SEQ ID NO:38); zebrafish nd4 forward mitoTALE nuclease, 5'-TATATTCGAAGCTAC-3' (SEQ ID NO:39); zebrafish nd4 reverse mitoTALE nuclease 5'-CGGGTAATAATGATT-3' (SEQ ID NO:40); zebrafish nd4 forward mitoTALE-nickase-1, 5'-TTACACCTAATTCCT-3' (SEQ ID NO:41); zebrafish nd4 reverse mitoTALE-nickase-1, 5'-AAGTTCTGGTTTTAA-3' (SEQ ID NO:42); zebrafish nd4 forward mitoTALE-nickase-2, 5'-GACTAAAATGAACCT-3' (SEQ ID NO:43); and zebrafish nd4 reverse mitoTALE-nickase-2, 5'-ACGAGTTAGAAGCAT-3' (SEQ ID NO:44). Once the RVDs were cloned into the GoldyTALE backbone, mRNA was generated for microinjections. Each nuclease or nickase expression plasmid was digested with *Sac*I for 2-3 hours at 37°C, and synthetic mRNA was generated (T3 mMessage mMachine kit, Ambion) and extracted with phenol/chloroform according to the manufacturer's instructions.

*Zebrafish mtDNA deletion screening:* 12.5 pg of each mitoTALE-nickase arm was microinjected in one-cell zebrafish embryos. An additional 50 pg of the mitoTALE nuclease was injected after the mitoTALE-nickase injection. After 3 and 5 days, individual larval zebrafish were collected in 1.7 mL microcentrifuge tubes, and DNA was extracted in a 40 µL of an extraction buffer (50 mM Tris-HCl pH 8.5, 1 mM EDTA, 0.5% Tween-20, 200 µg/ml proteinase K) at 55°C for 3-4 hours, vortexing once at 2 hours and centrifuging at 17,000xg for 1 minute after extraction was complete (Artuso et al., Biochim Biophys Acta 1817:1002-1011, 2012). One microliter of DNA extract was then added to a Platinum Taq PCR master mix (Invitrogen). For screening nd4 deletions, the following thermocycler parameters were used: 95°C for 5 minutes, (95°C for 30 seconds, 60°C for 30 seconds, 72°C for 1 minute) x 35, and 72°C for 5 minutes. The primers used were forward primer 5'-GGTCCGCCCGCCTACCATTTTC-3' (SEQ ID NO:45), reverse primer 5'-CCAGGTGATGAATAAGGCGATTGAGGT-3' (SEQ ID NO:46). For screening nd5/atp8 deletions, the following thermocycler parameters were used: 95°C for 5 minutes, (95°C for 30 seconds, 62°C for 30 seconds, 72°C for 1 minute) x 35, and 72°C for 5 minutes, and the primers used were forward primer 5'-GACGCGGTACCCGGACGATTAAACCA-3' (SEQ ID NO:47), reverse primer 5'-GCAATAATGCTTCCTCAGGCAAGCCGT-3' (SEQ ID NO:48). In both cases, 5-10 µL of PCR product was run on a 1.5-2% gel and extracted for sequencing using the Qiaex II gel extraction kit (Qiagen), then cloned using the TOPO PCR cloning kit for sequencing (Invitrogen).

*Long-Range PCR of Δnd5*/*atp8-mtDNA mutants:* One microliter of the individual larval DNA extract was run with standard MyTaq Red DNA polymerase (Bioline) in the company-supplied 5x buffer for a final reaction volume of 25 uL. The reaction was heated to 95°C for 10 minutes, and then cooled to 72°C and held until the polymerase was added (0.1 uL/reaction). The long-range PCR was then run with the following thermocycler parameters: 95°C for 5 minutes, (95°C for 30 seconds, 62°C for 30 seconds, 72°C for 8 minutes) x 35, and 72°C for 10 minutes. Ten (10) µL of the reaction was electrophoresed on a 0.8% agarose gel for imaging. The primers used were forward primer 5'-GACGCGGTACCCGGACGATTAAACCA-3' (SEQ ID NO:47), reverse primer 5'-GCAATAATGCTTCCTCAGGCAAGCCGT-3' (SEQ ID NO:48).

*Three-primer PCR for heteroplasmy quantification:* The protocol used for heteroplasmy assessment was adapted from a method described elsewhere (Sciacco et al., Hum. Mol. Genet. 3:13-19, 1994). This method uses three primers simultaneously in a PCR reaction to detect both the wild type and Δnd5/atp8-mtDNA in the same reaction, where they can be compared quantitatively. Two primers, forward primer 5'-GACGCGGTACCCGGACGATTAAACCA-3' (SEQ ID NO:47), and reverse primer 5'-GCAATAATGCTTCCTCAGGCAAGCCGT-3' (SEQ ID NO:48), flanked the predicted deletion site (125 pmoles each). The third reverse primer was located inside the deleted area, 5'-AACTCTTGGTGCAACTCCAAGTAGAAG-3' (SEQ ID NO:49) (31.25 pmoles). The two primers flanking the predicted deletion site were designed to only amplify the deleted genome (about 770 bp), while the reverse primer inside the deleted area and the forward primer outside would amplify wild type genomes (1421 bp). MyTaq Red DNA polymerase in the company-supplied 5x buffer was used for the PCR reaction to a final volume of 25 µL (Bioline). The reaction was heated to 95°C for 10 minutes, then cooled to 72°C and held until the polymerase was added (0.1 uL/reaction). The following run was executed in a thermocycler: 95°C for 5 minutes, (95°C for 15 seconds, 62°C for 15 seconds, 72°C for 1 minute) x 30, 72°C for 5 minutes. Ten (10) µL of the reaction was electrophoresed on a 1% agarose gel and the image was captured and band intensity was analyzed in Image J. The calculation for % heteroplasmy was (ΔmtDNA)/(ΔmtDNA + WT mtDNA).

*RT-PCR to measure relative mtDNA copy number:* The protocol used to measure mtDNA copy number in zebrafish was adapted from a method described elsewhere (Artuso et al., *supra).* The primers targeting mtDNA were in the *mt-nd1* gene (forward primer 5'-AGCCTACGCCGTACCAGTATT-3' (SEQ ID NO:50), reverse primer 5'-GTTTCACGCCATCAGCTACTG -3' (SEQ ID NO:51)), and the reference nuclear DNA primers were in the *polg* gene (forward primer 5'-GAGAGCGTCTATAAGGAGTAC-3' (SEQ ID NO:52), reverse primer 5'-gagctcatcagaaacaggact-3' (SEQ ID NO:53)). RT-PCR reactions consisted of the following: IX SensiFAST SYBR Lo-ROX mix (Bioline), 300 nM of the forward and reverse primer, 1 µL of the DNA extract and water to reach a final volume of 10 uL. This reaction was run in a CFX96 Touch Real-Time PCR Detection System (Biorad), and the cycling conditions were as follows: 95°C for 10 minutes, (95°C for 10 seconds, 55°C for 10 seconds, 72°C for 4 seconds) x 40, followed by a melting curve analysis to ensure the presence of only one PCR product (this was subsequently verified by running 5 µL of PCR product on a 1.5% agarose gel). Nuclear and mitochondrial DNA reactions were run in the same reaction to account for inter-run variability. For analysis, the nuclear DNA Ct value (the number of PCR cycles needed to cross the fluorescent threshold to distinguish signal from background during Real Time PCR; the value quantifies the amount of DNA the PCR primers are targeting in a sample) was subtracted from the mitochondrial DNA Ct value of individual samples to determine each sample's ΔCt. The ΔCt values from uninjected animals were averaged and subtracted from the calculated ΔCt of each sample to determine each sample's ΔΔCt. The fold change was then calculated (2-ΔΔCt) for each sample, and the fold change of each experimental group was averaged and the standard error determined. Samples with a Ct value over 23 after the polg (nuclear DNA) amplification were excluded as outliers (7 of 168 total samples). Two more samples were removed from the nickases and nuclease treated group as they were assumed to be outliers, with mtDNA fold change greater than 2. To measure statistical significance, a Student's t-test was performed on the fold change values unpaired, random values with unequal variances and non-Gaussian distribution. The scatter dot plot was prepared and statistical tests performed using GraphPad Prism software.

### Results

DSBs are known to trigger rapid mtDNA degradation and have been used strategically to combat the deleterious effects of heteroplasmy in patient cells and mouse models of mtDNA disease (Bayona-Bafaluy et al., Proc. Natl. Acad. Sci. USA 102:14392-14397, 2005; Alexeyev et al., Gene Ther. 15:516-523, 2008; Minczuk et al., *supra*; Reddy et al. Cell 161:459-469, 2015; and Bacman et al., Nat. Med. 19:1111-1113, 2013). To address this technical challenge, a system for targeted manipulation of mtDNA sequences was developed using editing strategies alternative to non-homologous end joining (NHEJ) and homology directed repair (HDR). Zebrafish was used as a rapid *in vivo* test system, because of its conserved mtDNA genome (Broughton et al., Genome Res. 11:1958-1967, 2001) and ease of microinjection for efficient and quantitative delivery of genome editing tools. TALE use in mtDNA engineering requires a single effective mitochondrial targeting sequence (FIG. 6), which was developed from an *in vivo* protein trapping method (Clark et al., Nat. Methods 8:506-515, 2011). This strong zebrafish mitochondrial targeting sequence was fused to the GoldyTALE backbone (Bedell et al. *supra)* in both nuclease and nickase forms, the latter made by mutagenizing half of the *Fok*I dimer to render it catalytically inactive (D450A23, FIG. 7A), with the hypothesis that the resulting mitoTALE-nickase would be able to directly manipulate the mtDNA genome to make single-gene deletions, as well as larger mtDNA mutations that molecularly model human disease.

Initial targeting of the 1.2 kb protein-encoding gene nd4 was used to test whether this approach could yield single-gene deletions for mitochondrial DNA functional testing. By positioning two mitoTALE-nickases just inside the border of the nd4 gene, deletions were readily detected by PCR in 50% of injected embryos (FIG. 7B). The PCR products were sequenced, confirming that mtDNA deletions were targeted to and near the mitoTALE-nickase sites (FIGS. 7C and 7D). nd4 was deleted using this approach in every case, with nearby DNA also being deleted (FIG. 7D).

A multitude of mtDNA diseases are caused by recurring multi-gene deletions. For example, a "common deletion" found in diseases such as Kearns-Sayre Syndrome (KSS) is a 4977 base-pair deletion located between nd5 and atp8 (Moraes et al., N. Engl. J. Med. 320:1293-1299, 1989; and Schon et al., Science 244:346-349, 1989). To molecularly model this disease, mitoTALE-nickases were positioned within these genes and deletions were detected by bridge-PCR and sequencing (FIG. 8A). The bridge-PCR was designed to selectively amplify deleted mtDNA (∼770 bp) by positioning two primers outside the mitoTALE-nickase sites and maintaining a short extension time low to avoid amplification of the full-length wild type product (5642 bp, FIG. 8A). Using this method, deletions were detected in nearly 30% of injected animals, and sequence verification confirmed deletion junctions near the mitoTALE-nickase targeted sites (designated the Δnd5/atp8-mtDNA genome, FIGS. 8B and 8C). The resulting deletions were relatively more precise compared to the nd4 deletions, with 5/10 of the sequenced deletion junctions within the mitoTALE-nickase binding sites or spacer (FIGS. 8C and 8D). Interestingly, the nd5 junction side of these deletions was more precise than the atp8 side (FIG. 8D).

Nucleases are effective at shifting mtDNA heteroplasmy by selectively targeting and cutting genomes that possess the nuclease-binding site (Hashimoto et al., Mol. Ther. 23:1592-1599, 2015), thereby driving their degradation. To determine whether this strategy could selectively target WT-mtDNA to preferentially enrich for the seeded Δnd5/atp8-mtDNA genomes induced by nickases, a full nuclease (mitoTALE nuclease) was designed against nd4, located between the nd5 and atp8 mitoTALE-nickases target sites in WT-mtDNA (FIG. 9A). Simultaneous delivery of the nd5 mitoTALE-nickase, atp8 mitoTALE-nickase, and nd4 mitoTALE nuclease resulted in over 90% of injected animals harboring deletions detectable using standard bridge-PCR conditions (FIG. 9B). These deletions were similar in sequence to the nickase-alone injected animals (FIG. 10). As a second detection system to complement bridge-PCR, long-range PCR was used to co-amplify wild type mtDNA along with any accompanying deletions (FIG. 9C). The long-range PCR resulted in amplification of WT-mtDNA in both injected and uninjected animals, while Δnd5/atp8-mtDNA genomes amplified only when mitoTALE-nickases were coinjected (FIG. 9C). Notably, the mutation-associated amplicons were amplified in greater abundance when the mitoTALE nuclease was injected as well (FIG. 9C). To quantify the heteroplasmy of mitoTALE-nickase-alone injected fish compared with those injected with both mitoTALE-nickases and the mitoTALE nuclease, a variation of three-primer PCR was used (Sciacco et al., Hum. Mol. Genet. 3:13-19, 1994) (FIG. 9A). By this approach, the percent heteroplasmy in mitoTALE-nickase-alone injected animals that were positive for bridge-PCR was indistinguishable from the percent heteroplasmy for uninjected animals (FIG. 9D), indicating a relatively low copy number of Δnd5/atp8-mtDNA genomes during the initial seeding process. However, animals additionally injected with a mitoTALE nuclease and positive for bridge-PCR had substantially increased heteroplasmy percentages (about 2% to about 10%; FIG. 9D). These data strongly suggested an increase in the number of Δnd5/atp8-mtDNA genomes compared to WT-mtDNA genomes after the mitoTALE nuclease was introduced. mtDNA depletion also was assessed, with a modest net loss (∼20%) after the nickases and nuclease were injected (FIG. 9E). Finally, long-term maintenance of the Δnd5/atp8-mtDNA genomes was assessed through fin-clipping biopsies of injected animals at 6 months post-injection. Deletions were present in 4/15 animals that had been injected with both mitoTALE-nickases and the mitoTALE nuclease, and the sequences were similar to those found in injected embryos (FIG. 9F).

Similar experiments were conducted with 293 cells, with DNA isolation and PCR analysis five days post transfection of A8 nickase, ND5 nickase, ND4 nuclease, A8 nickase and ND5 nickase together, or A8 nickase, ND5 nickase, and ND4 nuclease together. The nickases and/or nuclease were transfected into 293T cells using the 100 µL tip of a Neon electroporation system (Invitrogen). 100 ng of green fluorescent protein (GFP) was co-transfected with each condition to ensure efficient transfection. Transfections were done by delivery of 500 ng of A8 nickase, 500 ng of ND5 nickase, 1000 ng of ND4 nuclease, 500 ng of both A8 and ND5 nickase, or 500 ng of both A8 and ND5 nickases with 1000 ng of ND4 nuclease, or GFP alone. Cells were grown at 30°C for the first 20 hours post transfection, then moved to 37°C until samples were collected four days after transfection. The mtDNA was extracted using an mtDNA isolation kit (BioVision, Inc.). Each sample was diluted to 50 ng/uL, and PCR was performed using the MyTaq Red kit (Bioline) and run on a 1% agarose gel with to detect mtDNA deletions. PCR products were excised from the gel and extracted using the Qiaex II gel isolation kit (Qiagen), then cloned into a TOPO vector for sequencing (Invitrogen). Target sequences and primers were as follows.
A8 nickase forward target sequence: 5'-ATTCCTCATCACCCA-3' (SEQ ID NO:99)
A8 nickase reverse target sequence: 5'-AGGTGGTAGTTTGTG-3' (SEQ ID NO:100)
ND5 nickase forward target sequence: 5'-CTCACCCTAACAGGT-3' (SEQ ID NO:101)
ND5 nickase reverse target sequence: 5'-AATGTTAGTAAGGGT-3' (SEQ ID NO:102)
ND4 nuclease forward sequence: 5'-CACAATCATGGCAAG-3' (SEQ ID NO:103)
ND4 nuclease reverse sequence: 5'-GATAGTGGTTCACTG-3' (SEQ ID NO:104)
Forward primer: 5'-CTCATGAGCTGTCCCCACATTAGGCTT-3' (SEQ ID NO: 105)
Reverse primer: 5'-GCGATGGAGGTAGGATTGGTGCTGTGG-3' (SEQ ID NO:106)

PCR reactions included 1 µL 50ng/µL DNA (50 ng), 5 µL 5x Buffer for MyTaq Red, 0.5 µL 20 µM primer mix, 0.1 µL Taq (added when samples were placed in the thermocycler), and 18.4 µL water. Thermocycler conditions included 95°C for 10 minutes, 72°C on hold while Taq was added, (95°C for 30 seconds, 62°C for 30 seconds, 72°C for 1 minute) x 35, followed by 72°C for 5 minutes and a hold at 4°C until the gel was run. Ten (10) µL of each PCR reaction was run on a 1% agarose gel.

Deletions were detected by PCR only when A8 nickase, ND5 nickase, and ND4 nuclease were cotransfected (FIG. 11A). The PCR product was sequenced to confirm the location of a human mtDNA deletion close to each nick site. The dashes within the sequences shown in FIG. 11B indicate nucleotides that were deleted. Boxed sequences indicate the nickase sites (including the forward TAL site, reverse TAL site, and 15 bp spacer sequence). A gap of 119 bp is indicated on the A8 side to demonstrate how far the deletion junction is from the A8 nick site (FIG. 11B, bottom sequence).

The ability to precisely manipulate the mitochondrial genome is critical to understanding this complex organelle and to make more accurate disease models. Based on the findings discussed herein, a two-part approach to mitochondrial DNA engineering may be useful (FIG. 12). For part one, targeted mitoTALE-nickases are used to induce the desired seed deletion. The second component deploys a matched mitoTALE nuclease that targets the deleted region in the wild type genome to shift the heteroplasmy and expand the manipulated mtDNA genome. This can be accomplished both *in vitro* and *in vivo* through regulated expression of the mitoTALE nuclease subsequent to transient expression of the mitoTALE-nickases. Such an approach would enable the tunable production of cells with differential heteroplasmy levels. Similarly, complex *in vivo* animal models that recapitulate human disease (from zebrafish to mammals) can be developed through temporal or spatial (organ-specific) regulation of mitoTALE nucleases after a one-time delivery of mitoTALE-nickases to initiate targeted mtDNA deletions in an embryo, as conducted here in the zebrafish test system. Additionally, this system may avoid the need to pass manipulated mtDNA through the germline of animals to make severe disease models (Fan et al., Science 319:958-962, 2008), as mutations are made *de novo* and the resulting heteroplasmy is tunable.

### OTHER EMBODIMENTS

It is to be understood that while the invention has been described in conjunction with the detailed description thereof, the foregoing description is intended to illustrate and not limit the scope of the invention, which is defined by the scope of the appended claims.

## Claims

1. An *in vitro* method for modifying mitochondrial DNA within a cell, comprising
introducing into the cell a recombinant DNA endonuclease targeted to a selected sequence within the mitochondrial DNA, and
introducing into the cell a second recombinant DNA endonuclease targeted to a second selected sequence within the mitochondrial DNA, wherein the first DNA endonuclease and the second DNA endonuclease are targeted to different sequences within the mitochondrial DNA,
wherein the method is not a process for modifying the germ line genetic identity of human beings.

2. The method of claim 1, wherein the recombinant DNA endonuclease comprises a mutation that results in attenuated endonuclease activity, as compared to a corresponding DNA endonuclease that lacks the mutation.

3. The method of claim 1, wherein the recombinant DNA endonuclease has nickase activity.

4. The method of claim 1, wherein the recombinant DNA endonuclease comprises:
(a) a first portion comprising a mitochondrial targeting sequence;
(b) a second portion comprising an amino acid sequence targeting the DNA endonuclease to the selected sequence within the mitochondrial DNA; and
(c) a third portion comprising a nuclease domain that cleaves the mitochondrial DNA at or near the selected sequence, optionally wherein the first portion comprises a mitochondrial targeting sequence from isocitrate dehydrogenase 2,
optionally wherein the second portion comprises a transcriptional activator-like effector (TALE) backbone and a plurality of tandem repeat sequences comprising repeat variable dinucleotides that, in combination, are targeted to the selected mitochondrial DNA sequence.

5. The method of claim 4, wherein the nuclease domain is from a modified *Fok*I endonuclease, or a portion thereof, optionally wherein the modified *Fok*I endonuclease or portion thereof comprises an aspartic acid to alanine substitution at the amino acid position corresponding to position 483 of an unmodified *Fok*I endonuclease, or
wherein the modified *Fok*I endonuclease or portion thereof comprises an aspartic acid to alanine substitution at the amino acid position corresponding to position 450 of an unmodified *Fok*I endonuclease.

6. The method of claim 1, wherein the introducing comprises transforming a nucleic acid encoding the DNA endonuclease into the cell.

7. A recombinant polypeptide comprising:
(a) a first portion comprising a mitochondrial targeting sequence;
(b) a second portion comprising an amino acid sequence targeting the polypeptide to a selected mitochondrial DNA sequence; and
(c) a third portion comprising a nuclease domain that cleaves mitochondrial DNA at or near the selected sequence,
wherein the first portion comprises a mitochondrial targeting sequence from isocitrate dehydrogenase 2.

8. The polypeptide of claim 7, wherein the second portion comprises a TALE backbone and a plurality of tandem repeat sequences comprising repeat variable dinucleotides that, in combination, are targeted to the selected mitochondrial DNA sequence.

9. The polypeptide of claim 7, wherein the nuclease domain is from a modified *Fok*I endonuclease, or a portion thereof, optionally wherein the modified *Fok*I endonuclease or portion thereof comprises an aspartic acid to alanine substitution at the amino acid position corresponding to position 483 of an unmodified *Fok*I endonuclease, or
wherein the modified *Fok*I endonuclease or portion thereof comprises an aspartic acid to alanine substitution at the amino acid position corresponding to position 450 of an unmodified *Fok*I endonuclease.

10. A nucleic acid comprising a nucleotide sequence that encodes the polypeptide of claim 7.

11. A vector comprising the nucleic acid of claim 10.

12. A host cell containing the vector of claim 11, wherein the cell is not the human body, at the various stages of its formation and development.

## Patentansprüche

1. In-vitro-Verfahren zur Modifizierung mitochondrialer DNA in einer Zelle, umfassend
Einführen einer rekombinanten DNA-Endonuklease, die auf eine ausgewählte Sequenz in der mitochondrialen DNA gerichtet ist, in die Zelle und
Einführen einer zweiten rekombinanten DNA-Endonuklease, die auf eine zweite ausgewählte Sequenz in der mitochondrialen DNA gerichtet ist, in die Zelle, wobei die erste DNA-Endonuklease und die zweite DNA-Endonuklease auf unterschiedliche Sequenzen in der mitochondrialen DNA gerichtet sind,
wobei es sich bei dem Verfahren nicht um einen Prozess zur Veränderung der genetischen Identität der Keimbahn des Menschen handelt.

2. Verfahren nach Anspruch 1, wobei die rekombinante DNA-Endonuklease eine Mutation umfasst, die zu abgeschwächter Endonuklease-Aktivität im Vergleich zu einer entsprechenden DNA-Endonuklease, der die Mutation fehlt, führt.

3. Verfahren nach Anspruch 1, wobei die rekombinante DNA-Endonuklease Nickase-Aktivität aufweist.

4. Verfahren nach Anspruch 1, wobei die rekombinante DNA-Endonuklease Folgendes umfasst:
(a) einen ersten Teil, der eine mitochondriale Targetingsequenz umfasst;
(b) einen zweiten Teil, der eine Aminosäuresequenz umfasst, die die DNA-Endonuklease auf die ausgewählte Sequenz in der mitochondrialen DNA richtet; und
(c) einen dritten Teil, der eine Nuklease-Domäne umfasst, die die mitochondriale DNA an oder nahe der ausgewählten Sequenz spaltet, gegebenenfalls wobei der erste Teil eine mitochondriale Targetingsequenz aus Isocitrat-Dehydrogenase 2 umfasst,
gegebenenfalls wobei der zweite Teil ein TALE (Transcriptional Activator-Like Effector)-Grundgerüst und mehrere Tandem-Repeat-Sequenzen umfasst, die wiederholte variable Dinukleotide umfassen, die zusammen auf die ausgewählte mitochondriale DNA-Sequenz gerichtet sind.

5. Verfahren nach Anspruch 4, wobei die Nuklease-Domäne aus einer modifizierten *Fok*I-Endonuklease oder einem Teil davon stammt, gegebenenfalls wobei die modifizierte *Fok*I-Endonuklease oder der Teil davon eine Asparaginsäure-zu-Alanin-Substitution an der Aminosäureposition, die Position 483 einer unmodifizierten *Fok*I-Endonuklease entspricht, umfasst, oder
wobei die modifizierte *Fok*I-Endonuklease oder der Teil davon eine Asparaginsäure-zu-Alanin-Substitution an der Aminosäureposition, die Position 450 einer unmodifizierten *Fok*I-Endonuklease entspricht, umfasst.

6. Verfahren nach Anspruch 1, wobei das Einführen Transformieren einer die DNA-Endonuklease codierenden Nukleinsäure in die Zelle umfasst.

7. Rekombinantes Polypeptid, umfassend:
(a) einen ersten Teil, der eine mitochondriale Targetingsequenz umfasst;
(b) einen zweiten Teil, der eine Aminosäuresequenz umfasst, die das Polypeptid auf eine ausgewählte mitochondriale DNA-Sequenz richtet; und
(c) einen dritten Teil, der eine Nuklease-Domäne umfasst, die mitochondriale DNA an oder nahe der ausgewählten Sequenz spaltet,
wobei der erste Teil eine mitochondriale Targetingsequenz aus Isocitrat-Dehydrogenase 2 umfasst.

8. Polypeptid nach Anspruch 7, wobei der zweite Teil ein TALE-Grundgerüst und mehrere Tandem-Repeat-Sequenzen umfasst, die wiederholte variable Dinukleotide umfassen, die zusammen auf die ausgewählte mitochondriale DNA-Sequenz gerichtet sind.

9. Polypeptid nach Anspruch 7, wobei die Nuklease-Domäne aus einer modifizierten *Fok*I-Endonuklease oder einem Teil davon stammt, gegebenenfalls wobei die modifizierte *Fok*I-Endonuklease oder der Teil davon eine Asparaginsäure-zu-Alanin-Substitution an der Aminosäureposition, die Position 483 einer unmodifizierten *Fok*I-Endonuklease entspricht, umfasst, oder
wobei die modifizierte *Fok*I-Endonuklease oder der Teil davon eine Asparaginsäure-zu-Alanin-Substitution an der Aminosäureposition, die Position 450 einer unmodifizierten *Fok*I-Endonuklease entspricht, umfasst.

10. Nukleinsäure, umfassend eine Nukleotidsequenz, die das Polypeptid nach Anspruch 7 codiert.

11. Vektor, umfassend die Nukleinsäure nach Anspruch 10.

12. Wirtszelle, enthaltend den Vektor nach Anspruch 11, wobei es sich bei der Zelle nicht um den menschlichen Körper auf den verschiedenen Stufen seiner Bildung und Entwicklung handelt.

## Revendications

1. Procédé *in vitro* destiné à modifier l'ADN mitochondrial au sein d'une cellule, comprenant les étapes consistant à
introduire dans la cellule une endonucléase d'ADN recombinante ayant pour cible une séquence choisie au sein de l'ADN mitochondrial et
introduire dans la cellule une deuxième endonucléase d'ADN recombinante ayant pour cible une deuxième séquence choisie au sein de l'ADN mitochondrial, dans lequel la première endonucléase d'ADN et la deuxième endonucléase d'ADN ont pour cible différentes séquences au sein de l'ADN mitochondrial,
dans lequel le procédé n'étant pas un processus destiné à modifier l'identité génétique de la lignée germinale d'êtres humains.

2. Procédé de la revendication 1, dans lequel l'endonucléase d'ADN recombinante comprend une mutation qui conduit à une activité atténuée d'endonucléase, en comparaison avec une endonucléase d'ADN correspondante dépourvue de la mutation.

3. Procédé de la revendication 1, dans lequel l'endonucléase d'ADN recombinante a une activité de nickase.

4. Procédé de la revendication 1, dans lequel l'endonucléase d'ADN recombinante comprend :
(a) une première partie comprenant une séquence de ciblage mitochondrial ;
(b) une deuxième partie comprenant une séquence d'acides aminés faisant que l'endonucléase d'ADN cible la séquence choisie au sein de l'ADN mitochondrial ; et
(c) une troisième partie comprenant un domaine de nucléase qui coupe l'ADN mitochondrial au niveau de la séquence choisie ou près de celle-ci, en option dans lequel la première partie comprend une séquence de ciblage mitochondrial provenant d'une isocitrate déshydrogénase 2,
en option dans lequel la deuxième partie comprend un squelette d'effecteur du type activateur de la transcription (TALE) et une pluralité de séquences de répétition en tandem comprenant des dinucléotides variables répétitifs lesquels, en combinaison, ont pour cible la séquence choisie de l'ADN mitochondrial.

5. Procédé de la revendication 4, dans lequel le domaine de nucléase provient d'une endonucléase *FokI* modifiée ou d'une partie de celle-ci, en option dans lequel l'endonucléase *FokI* modifiée, ou une partie de celle-ci, comprend un remplacement d'un acide aspartique par une alanine au niveau de la position d'acide aminé correspondant à la position 483 d'une endonucléase *FokI* non modifiée ou
dans lequel l'endonucléase *FokI* modifiée, ou une partie de celle-ci, comprend un remplacement d'un acide aspartique par une alanine au niveau de la position d'acide aminé correspondant à la position 450 d'une endonucléase *FokI* non modifiée.

6. Procédé de la revendication 1, dans lequel l'introduction comprend une transformation dans la cellule d'un acide nucléique codant pour l'endonucléase d'ADN.

7. Polypeptide recombinant comprenant :
(a) une première partie comprenant une séquence de ciblage mitochondrial ;
(b) une deuxième partie comprenant une séquence d'acides aminés faisant que le polypeptide cible une séquence choisie de l'ADN mitochondrial ; et
(c) une troisième partie comprenant un domaine de nucléase qui coupe l'ADN mitochondrial au niveau de la séquence choisie ou près de celle-ci,
dans lequel la première partie comprend une séquence de ciblage mitochondrial provenant d'une isocitrate déshydrogénase 2.

8. Polypeptide de la revendication 7, dans lequel la deuxième partie comprend un squelette de TALE et une pluralité de séquences de répétition en tandem comprenant des dinucléotides variables répétitifs lesquels, en combinaison, ont pour cible la séquence choisie de l'ADN mitochondrial.

9. Polypeptide de la revendication 7, dans lequel le domaine de nucléase provient d'une endonucléase *Fok*I modifiée ou d'une partie de celle-ci, en option dans lequel l'endonucléase *Fok*I modifiée, ou une partie de celle-ci, comprend un remplacement d'un acide aspartique par une alanine au niveau de la position d'acide aminé correspondant à la position 483 d'une endonucléase *Fok*I non modifiée ou
dans lequel l'endonucléase *Fok*I modifiée, ou une partie de celle-ci, comprend un remplacement d'un acide aspartique par une alanine au niveau de la position d'acide aminé correspondant à la position 450 d'une endonucléase *Fok*I non modifiée.

10. Acide nucléique comprenant une séquence nucléotidique qui code pour le polypeptide de la revendication 7.

11. Vecteur comprenant l'acide nucléique de la revendication 10.

12. Cellule hôte contenant le vecteur de la revendication 11, la cellule n'étant pas le corps humain, aux divers stades de sa formation et de son développement.
